# EUROPEAN PATENT APPLICATION

(11) **EP 2 082 740 A2**
(43) Date of publication of application: **29.07.2009**
(21) Application number: 09004628.5
(22) Date of filing: 06.07.1998
(51) Int. Cl.: A61K 31/4164, A61K 31/4178, A61K 31/42, A61K 31/423, A61K 31/428, A61K 31/454, A61K 31/497, A61K 31/5377, A61K 31/496

(54) **Use of imidazole derivatives as MDR modulators**

(30) Priority: 09.07.1997 US 890911
(62) Divisional of application: 98934267.0
(71) Applicant: Taiji Biomedical, Inc., Menlo Park, CA 94025 (US)
(72) Inventor: Mjalli, Adnan, Louisville, KY 40241 (US); Zhang, Chengzhi, Carlsbad, CA 92009 (US)
(74) Representative: Sauvage, Renée

(57) **Abstract**

The invention relates to the use of imidazole derivatives having formula (I) 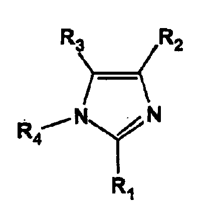 or their pharmaceutically acceptable salts for treating tumor cells and for increasing the sensitivity of multidrug resistant tumor cells to antitumor chemotherapeutic agents.

## Description

### FIELD OF THE INVENTION

The present invention provides novel imidazole derivatives, novel pharmaceutical compositions containing same, and medical uses

Such compounds are pharmacologically useful for restoring the sensitivity of multidrug resistant cells to cancer chemotherapeutic agents.

### BACKGROUND OF THE INVENTION

A major problem in the treatment of cancer is the emergence of tumor cell resistance to chemotherapeutic agents and the subsequent patient relapse (Bradley et al.I, Cancer Res. 1989, 49, 2790-2796; Raderer and Sscheitharer, Cancer 1993, 72, 3553-3563). These cancer victims may fail to respond to any antitumor agent, since these tumor cells tend to exhibit clinical resistance to many drugs. This phenomenon is known as multi-drug resistance (MDR). MDR is associated with certain alterations in tumor cells resulting in reduced intracellular anticancer drug accumulation, including reduced membrane permeability and increased removal of drug from the cell *via* an energy-dependent efflux mechanism. Studies of this mechanism have led to the characterization of genes capable of conferring resistance to chemotherapeutic agents. One of these genes, the P-glycoprotein or MDR1 gene, has been strongly implicated since overexpression of this gene can lead to resistance to anthracyclines, vinca alkaloids, and podophyllins, all important chemotherapeutic agents. MDR1 encodes a 170 kDa membrane glycoprotein (gp-170 or Pgp) that acts as an ATP-dependent dfflux pump, transporting a number of unrelated organic compounds out of the cell (Juranka et al, FASEB J. 1989, 3, 2583-2592). The level of expression of gp-170 has been shown to correlate with the degree of drug resistance (Raderer and Sscheitharer, Cancer 1993, 72, 3553-3563). Gp-170 appears to act as a pump that actively extrudes a wide variety of structurally unrelated compounds, including a full range of antineoplastic drugs. Another ATP-dependent menbrane efflux pump, the product of the MRP gene, has also been implicated in the MDR phenomenon (Krishnamachary and Center, Cancer Res. 1993, 53, 3658-3661), as have other ATP-dependent and enzymatic mechanisms.

Drugs of proven antitumor chemotherapeutic value to which MDR has been observed include vinblastine, vincristine, etoposide, teniposide, doxorubicin (adriamycin), daunorubicin, pliamycin (mithramycin), and actinomycin D (Jones et al., Cancer (Suppl)1993, 72, 3484-3488). Many tumors are intrinsically multi-drug resistant (e.g., adenocarcinomas of the colon and kidney) while other tumors acquire MDR during the course of therapy (e.g., neuroblastomas and childhood leukemias).

A variety of structurally diverse agents have been identified which can restore partly or sometimes completely the normal drug sensitivity to some MDR tumor cells. It is assumed that these chemosensitizers are effective as a result of their ability to interfere with gp-170, causing a reversal in the increase in drug efflux. Among these agents are calcium channel blockers (e.g., verapamil), calmodulin inhibitors (*e.g.*, trifluoperazine), antibiotica (e.g., erythromycin), cardiovascularagents (*e.g.*, quinidine), noncytotoxic analogs of anthracyclines and vinca alkaloids, cyclosporin A and analogs thereof, FK-506 and analogs thereof, and derivatives of cyclopeptides (Lum et al.,Cancer (Suppl)1993, 72, 3502-3514). However, at the present time, none of these agents has provided a significant contribution to the chemotherapeutic index for the treatment of cancer due to their significant pharmacologidal effects on other organ systems. An effective therapeutic agent for the reversal of MDR needs to have efficacy against the menbrane pump as well as lack of significant toxicity and other nonspecific pharmacological effects.

The present invention describes a family of novel substituted imidazole derivatives of Formula (1) that are effective in increasing the sensitivity of tumor cells resistant to anticancer chemotherapeutic agents, such as doxorubicin (DOX), taxol, vinblastine (VLB), and enhancing the sensitivity of multi-drug resistant cells. These compounds have the effect of reducing the resistance of MDR tumor cells, and potentiating the sensitivity of cells to antitumor drugs, such as DOX, taxol, VLB. These compounds are expected to have broad application in the chemotherapy of cancer.

### SUMMARY OF THE INVENTION

The novel compounds of this invention have the general structure (1) 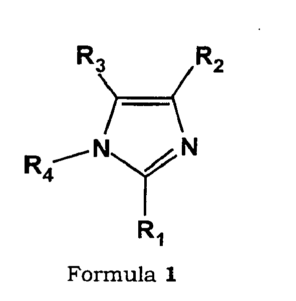 and are capable of restoring sensitivity to multi-drug resistant tumor cells. It is an object of this invention to provide compounds that have sufficient activity to sensitize multi-drug resistant tumor cells to antineoplastic agents.

It is an additional object of this invention to provide medicoments comprising the compounds of the invention for sensitizing multi-drug resistant tumor cells

A further object is to provide medicoments comprising a therapeutically effective of a compound of the present invention for the treatment of MDR or drug-sensitive tumor cells to be administered prior to, together with, or subsequent to the administration of an antitumor chemotherapeutic agent. A further object is to provide pharmaceutical compositions for increasing the sensitivity of tumor cells to antitumor chemotherapeutic agents and thus for the treatment of tumors that are susceptible to anti-cancer chemotherapeutic agents but have become resistant to such chemotherapy.

### Definitions

As used herein, the term "attached" signifies a stable covalent bond, certain preferred points of attachment being apparent to those skilled in the art.

The terms "halogen" or "halo" include fluorine, chlorine, bromine, and iodine.

The term "alkyl" includes C₁-C₁₁ straight chain saturated, C₁-C₁₁ branched saturated, C₃-C₈ cyclic saturated and C₁-C₁₁ straight chain or branched saturated aliphatic hydrocarbon groups substituted with C₃-C₈ cyclic saturated aliphatic hydrocarbon groups having the specified number of carbon atoms. For example, this definition shall include but is not limited to methyl (Me), ethyl (Et), propyl (Pr), butyl (Bu), pentyl, hexyl, heptyl, octyl, nonyl, decyl, undecyl, isopropyl (i-Pr), isobutyl (i-Bu), *tert*-butyl (t-Bu), *sec-*butyl (s-Bu), isopentyl, neopentyl, cyclopropyl, cyclobutyl, cyclopentyl, cyclohexyl, cycloheptyl, cyclooctyl, methylcyclopropyl, and the like.

The term "alkenyl" includes C₂-C₁₁ straight chain unsaturated, C₂-C₁₁ branched unsaturated, C₅-C₈ unsaturated cyclic, and C₂-C₁₁ straight chain or branched unsaturated aliphatic hydrocarbon groups substituted with C₃-C₈ cyclic saturated and unsaturated aliphatic hydrocarbon groups having the specified number of carbon atoms. Double bonds may occur in any stable point along the chain and the carbon-carbon double bonds may have either the cis or trans configuration. For example, this definition shall include but is not limited to ethenyl, propenyl, butenyl, pentenyl, hexenyl, heptenyl, octenyl, nonenyl, decenyl, undecenyl, 1,5-octadienyl, 1,4,7-nonatrienyl, cyclopentenyl, cyclohexenyl, cycloheptenyl, cyclooctenyl, ethylcyclohexenyl, butenylcyclopentyl, 1-pentenyl-3-cyclohexenyl, and the like.

The term "alkyloxy" (e.g. methoxy, ethoxy, propyloxy, allyloxy, cyclohexyloxy) represents an alkyl group as defined above having the indicated number of carbon atoms attached through an oxygen bridge.

The term "alkylthio" (e.g. methylthio, ethylthio, propylthio, cyclohexylthio and the like) represents an alkyl group as defined above having the indicated number of carbon atoms attached through a sulfur bridge.

The term "alkylamino" (e.g. methylamino, diethylamino, butylamino, N-propyl-N-hexylamino, (2-cyclopentyl)propylamino, hexylamino, pyrrolidinyl, piperidinyl and the like) represents one or two alkyl groups as defined above having the indicated number of carbon atoms attached through an amine bridge. The two alkyl groups maybe taken together with the nitrogen to which they are attached forming a cyclic system containing 3 to 11 carbon atoms with at least one C₁-C₁₁aloyl, arylC₀-C₁₁alkyl substituent.

The term "alkylaminoalkyl" represents an alkylamino group attached through an alkyl group as defined above having the indicated number of carbon atoms.

The term "alkyloxy(alkyl)amino" (e.g. methoxy(methyl)amine, ethoxy(propyl)amine) represents an alkyloxy group as defined above attached through an amino group, the amino group itself having an alkyl substituent.

The term "alkylcarbonyl" (e.g. cyclooctylcarbonyl, pentylcarbonyl, 3-hexylcarbonyl) represents an alkyl group as defined above having the indicated number of carbon atoms attached through a carbonyl group.

The term "alkylcarboxy" (e.g. heptylcarboxy, cyclopropylcarboxy, 3-pentenylcarboxy) represents an alkylcarbonyl group as defined above wherein the carbonyl is in turn attached through an oxygen. The term "alkylcarboxyalkyl" represents an alkylcarboxy group attached through an alkyl group as defined above having the indicated number of carbon atoms.

The term "alkylcarbonylamino" (e.g. hexylcarbonylamino, cyclopentylcarbonyl-aminomethyl, methylcarbonylaminophenyl) represents an alkylcarbonyl group as defined above wherein the carbonyl is in turn attached through the nitrogen atom of an amino group. The nitrogen group may itself be substituted with an alkyl or aryl group.

The term "aryl" represents an unsubstituted, mono-, di- or trisubstituted monocyclic, polycyclic, biaryl and heterocyclic aromatic groups covalently attached at any ring position capable of forming a stable covalent bond, certain preferred points of attachment being apparent to those skilled in the art (e.g., 3-indolyl, 4-imidazolyl). The aryl substituents are independently selected from the group consisting of halo, nitro, cyano, trihalomethyl, C₁₋₁₁alkyl, arylC₁₋₁₁alkyl, C₀₋₁₁alkyloxyC₀₋₁₁alkyl, arylC₀₋₁₁alkyloxyC₀₋₁₁alkyl, C₀₋₁₁alkylthioC₀₋₁₁alkyl, arylC₀₋₁₁alkylthioC₀₋₁₁alkyl, C₀₋₁₁alkylaminoC₀₋₁₁alkyl, arylC₀₋₁₁alkylaminoC₀₋₁₁alkyl, di(arylC₁₋₁₁alkyl)aminoC₀₋₁₁alkyl, C₁₋₁₁alkylcarbonylC₀₋₁₁alkyl, arylC₁₋₁₁alkylcarbonylC₀₋₁₁alkyl, C₁₋₁₁alkylcarboxyC₀₋₁₁alkyl, arylC₁₋₁₁alkylcarboxyC₀₋₁₁alkyl, C₁₋₁₁alkylcarbonylaminoC₀₋₁₁alkyl, arylC₁₋₁₁alkylcarbonylaminoC₀₋₁₁alkyl, -C₀₋₁₁alkylCOOR₁, -C₀₋₁₁alkylCONR₂R₃ wherein R₁, R₂ and R₃ are independently selected from hydrogen, C₁-C₁₁alkyl, arylC₀-C₁₁alkyl, or R₂ and R₃ are taken together with the nitrogen to which they are attached forming a cyclic system containing 3 to 8 carbon atoms with at least one C₁-C₁₁alkyl, arylC₀-C₁₁alkyl substituent.

The definition of aryl includes but is not limited to phenyl, biphenyl, naphthyl, dihydronaphthyl, tetrahydronaphthyl, indenyl, indanyl, azulenyl, anthryl, phenanthryl, fluorenyl, pyrenyl, thienyl, benzothienyl, isobenzothienyl, 2,3-dihydrobenzothienyl, furyl, pyranyl, benzofuranyl, isobenzofuranyl, 2,3-dihydrobenzofuranyl, pyrrolyl, indolyl, isoindolyl, indolizinyl, indazolyl, imidazolyl, benzimidazolyl, pyridyl, pyrazinyl, pyradazinyl, pyrimidinyl, triazinyl, quinolyl, isoquinolyl, 4H-quinolizinyl, cinnolinyl, phthalazinyl, quinazolinyl, quinoxalinyl, 1,8-naphthyridinyl, pteridinyl, carbazolyl, acridinyl, phenazinyl, phenothiazinyl, phenoxazinyl, chromanyl, benzodioxolyl, piperonyl, purinyl, pyrazolyl, triazolyl, tetrazolyl, thiazolyl, isothiazolyl, benzthiazolyl, oxazolyl, isoxazolyl, benzoxazolyl, oxadiazolyl, thiadiazolyl.

The term "arylalkyl" (e.g. (4-hydroxyphenyl)ethyl, (2-aminonaphthyl)hexyl, pyridylcyclopentyl) represents an aryl group as defined above attached through an alkyl group as defined above having the indicated number of carbon atoms.

The term "carbonyloxy" represents a carbonyl group attached through an oxygen bridge.

In the above definitions, the terms "alkyl" and "alkenyl" maybe used interchangeably in so far as a stable chemical entity is formed, as obvious to those skilled in the art.

The term "therapeutically effective amount" shall mean that amount of drug or pharmaceutical agent that will elicit the biological or medical response of a tissue, system, animal, or human that is being sought by a researcher, veterinarian, medical doctor or other clinician.

### DETAILED DESCRIPTION OF THE INVENTION

The novel compounds of this invention have the general structure as depicted in Formula (1) 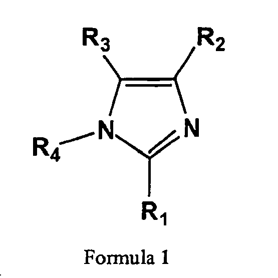 wherein the substituents R₁, R₂, R₃, and R₄ are defined as described in **A** and **B** in claim 1 or 2

Novel compounds of the present invention include but are not limited to the following compounds:
2-[trans-2-(2-benzoxazolyl)ethenylphenyl]-4,5-bis (4-N,N-dimethylaminophenyl) imidazole,
2-[trans-2-(2-benzoxazolyl)ethenylphenyl]-4-(4-N,N-dimethylaminophenyl)-5-(4-N-methylaminophenyl) imidazole,

2-[trans-2-(2-benzoxazolyl)ethenylphenyl]-4-(4-N,N-diethylaminophenyl)-5-(4-N-methylaminophenyl) imidazole,
2-[trans-2-(2-benzoxazolyl)ethenylphenyl]-4-(4-N-disopropylaminophenyl)-5-(4-N-methylaminophenyl) imidazole,

2-[trans-2-(2-benzoxazolyl)ethenylphenyl]-4-(4-N-methylaminophenyl)-5-(4-pyrrolidinophenyl) imidazole,
2-[trans-2-(2-benzoxazolyl)ethenylphenyl]-4-(4-N,N-dimethylaminophenyl)-5-[4-(2-methoxyethylamino)phenyl] imidazole,
2-[trans-2-(2-benzthiazolyl)ethenylphenyl]-4,5-bis (4-N,N-dimethylaminophenyl) imidazole,
2-(trans-2-(2-benzthiazolyl)ethenylphenyl]-4-(4-N,N-dimethylaminophenyl)-5-(4-N-methylaminophenyl) imidazole,

2-[trans-2-(2-cyano)ethenylphenyl]-4,5-(4-N,N-dimethylaminophenyl) imidazole,
2-[trans-2-(2-cyano)ethenylphenyl]-4-(4-N,N-dimethylaminophenyl)-5-[4-N-(2-methoxyethyl)amino)phenyl] imidazole,
2-(trans-2-methoxycarbonyl-ethenylphenyl)-4-(4-N,N-diallylaminophenyl)-5-(4-fluoro-phenyl) imidazole,
2-(trans-2-methoxycarbonyl-ethenylphenyl)-4-(4-N-methylaminophenyl)-5-(4-pyrrolidinophenyl) imidazole,
2-(trans-2-methoxycarbonyl-ethenylphenyl)-4-(4-N-methylaminophenyl)-5-(4-piperidinophenyl) imidazole,
2-(trans-2-methoxycarbonyl-ethenylphenyl)-4-[4-N,N-di(2-methoxyethyl)aminophenyl]-5-(4-N-methylaminophenyl) imidazole,
2-(trans-2-methoxycarbonyl-ethenylphenyl)-4-[4-(1-imidazolyl)phenyl]-5-(4-N-methylaminophenyl) imidazole,
2-(trans-2-methoxycarbonyl-ethenylphenyl)-4,5-bis (4-N-morpholinophenyl) imidazole,
2-(trans-2-methoxycarbonyl-ethenylphenyl)-4-(4-N,N-dimethylaminophenyl)-5-(4-N-morpholinophenyl) imidazole,
2-(trans-2-methoxycarbonyl-ethenylphenyl)-4-(4-N-methylaminophenyl)-5-(4-N-morpholinophenyl) imidazole:
2-[4-(3-methoxy-trans-1-propen-1-yl)phenyl]-4,5-bis (4-N,N-dimethylaminophenyl) imidazole,
2-[4-(3-ethoxy-trans-1-propen-1-yl)phenyl]-4,5-bis (4-N,N-dimethylaminophenyl) imidazole,
2-[4-(3-ethoxy-trans-1-propen-1-yl)phenyl]-4-(4-N,N-dimethylaminophenyl)-5-(4-N-methylaminophenyl) imidazole,
2-[4-(3-benzyloxy-trans-1-propen-1-yl)phenyl]-4,5-bis (4-N,N-dimethylaminophenyl) imidazole,
2-[4-(3-phenoxy-trans-l-propen-1-yl)phenyl]-4-(4-N,N-dimethylaminophenyl)-5-(4-N-methylaminopheny) imidazole,
2-{4-[3-(3,4-dimethoxy-phenoxy)-trans-1-propen-1-yl]phenyl}-4-(4-N,N-dimethylaminophenyl)-5-(4-N-methylaminopheny) imidazole,
2-{4-(3-N,N-diethylamino-trans-l-propen-l-yl)phenyl]-4,5-bis (4-N,N-dimethylaminophenyl) imidazole,
2-[4-(3-N-morpholino-trans-1-propen-1-yl)phenyl]-4,5-bis (4-N,N-dimethylaminophenyl) imidazole,
2-[4-(3-N-piperidino-trans-1-propen-1-yl)phenyl]-4,5-bis (4-N,N-dimethylaminophenyl) imidazole,
2-[4-(3-N,N-dimethylamino-trans-1-propen-1-yl)phenyl]-4,5-bis (4-N,N-dimethylaminophenyl) imidazole,
2-{4-[3-(2-methoxy-ethoxy)-trans-1-propen-1-yl]phenyl}-4,5-bis (4-N,N-dimethylaminophenyl) imidazole,
2-[4-(3-butoxy-trans-1-propen-l-yl)phenyl]-4,5-bis (4-N,N-dimethylaminophenyl) imidazole,
2-[4-(3-ethoxy-trans-1-propen-1-yl)phenyl]-4,5-bis (4-N,N-diethylaminophenyl) imidazole,
2-[4-(3-ethoxy-trans-1-propen-1-yl)phenyl)-4-(4-N,N-diethylaminophenyl)-5-(4-N-methylaminophenyl) imidazole,
2-[4-(3-methoxy-trans-1-propen-1-yl)phenyl)-4,5-bis (4-pyrrolidinophenyl) imidazole,
2-[4-(3-ethoxy-trans-1-propen-1-yl)phenyl]-4,5-bis (4-pyrrolidinophenyl) imidazole,
2-[4-(3-ethoxy-trans-1-propen-1-yl)phenyl)-4-(4-N,N-dimethylaminophenyl)-5-(4-pyrrolidinophenyl) imidazole,
2-[4-(3-ethoxy-trans-1-propen-1-yl)phenyl]-4-(4-N-methylaminophenyl)-5-(4-pyrrolidinophenyl) imidazole,
2-[4-(3-ethoxy-trans-1-propen-1-yl)phenyl]-4,5-bis (4-N-morpholinophenyl) imidazole,
2-[4-(3-ethoxy-trans-1-propen-1-yl)phenyl)-4-(4-N,N-dimethylaminophenyl)-5-(4-N-morpholinophenyl) imidazole,
2-[4-(3-ethoxy-trans-1-propen-1-yl)phenyl]-4-(4-N-methylaminophenyl)-5-(4-N-morpholinophenyl) imidazole,
2-[4-(3-ethoxy-trans-1-propen-1-yl)phenyl]-4-(4-N-methylaminophenyl)-5-(4-N-isopropylaminophenyl) imidazole,
2-[4-trans-(2-methanesulfonyl-ethenyl)-phenyl]-4,5-bis (4-N,N-dimethylaminophenyl) imidazole,
2-(4-N-morpholinophenyl)-4,5-bis (4-N,N-dimethylaminophenyl) imidazole,
2-[4-(5-ethylcarboxyisoxazol-3-yl)-phenyl]-4,5-bis (4-N,N-dimethylaminophenyl) imidazole,
2-[4-trans-(2-methoxycarbonyl-ethenyl)phenyl]-4-(p-tolyl)-5-(4-N,N-diethylaminomethylphenyl) imidazole,
2-[4-trans-(2-methoxycarbonyl-ethenyl)phenyl]-4,5-bis (4-N,N-diethylaminomethylphenyl) imidazole,
2-[4-trans-(2-methoxycarbonyl)cyclopropan-1-yl]-4,5-bis (4-N,N-diethylaminomethylphenyl) imidazole,
2-[4-(3-ethoxy-trans-1-propen-1-yl)phenyl]-4,5-bis (4-dimethoxyphenyl) imidazole,
2-[4-(3-ethoxy-trans-1-propen-1-yl)phenyl]-4,5-bis (4-diethoxyphenyl) imidazole,
2-[4-(3-ethoxy-trans-1-propen-1-yl)phenyl]-4,5-bis (4-diisopropyloxyphenyl) imidazole,
1-(3-imidazol-1-yl-propyl)-2-[4-(3-ethoxy-trans-1-propen-1-yl)phenyl]-4,5-bis (4-dimethoxyphenyl) imidazole,
1-(3-imidazol-1-yl-propyl)-2-[4-(3-ethoxy-trans-1-propen-1-yl)phenyl]-4,5-bis (4-diethoxyphenyl) imidazole,
1-(3-imidazol-1-yl-propyl)-2-[4-(3-ethoxy-trans-1-propen-1-yl)phenyl]-4,5-bis (4-diisopropyloxyphenyl) imidazole,
2-[4-(3-ethoxy-trans-1-propen-1-yl)phenyl]-4-(4-N,N-diethylphenyl) imidazole,
2-[4-(3-ethoxy-trans-1-propen-1-yl)phenyl]-4-(4-methoxyphenyl) imidazole,
2-[4-trans-(2-N,N-dimethylcarbonyl)-ethenyl]phenyl}-4,5-bis (4-N,N-dimethylaminophenyl) imidazole,
2-[4-(3-hydroxy-trans-1-propen-1-yl)phenyl]-4,5-bis (4-N,N-dimethylaminophenyl) imidazole,
1-methyl-2-[4-(3-hydroxy-trans-1-propen-1-yl)phenyl]-4,5-bis (4-N,N-dimethylaminophenyl) imidazole,
2-[4-(3-pivalate-trans-1-propen-1-yl)phenyl]-4,5-bis (4-N,N-dimethylaminophenyl) imidazole,
2-[4-(3-methylcarbonyl-trans-1-propen-1-yl)phenyl]-4,5-bis (4-N,N-dimethylaminophenyl) imidazole,
2-[4-(3-methylcarbonyl-trans-1-propen-1-yl)phenyl]-5-methoxy benzimidazole,
2-[4-(3-ethoxy-trans-1-propen-1-yl)phenyl]-4,5-bis-(4-N-isopropylaminophenyl) imidazole,
2-[4-(3-ethoxy-trans-1-propen-1-yl)phenyl]-4-(4-N-ethylaminophenyl)-5-(4-N-isopropylaminophenyl) imidazole,
2-[4-(3-ethoxy-trans-1-propen-1-yl)phenyl]-4-(4-fluorophenyl)-5-(4-N-isopropylaminophenyl) imidazole,
2-[4-(3-ethoxy-trans-1-propen-1-yl)phenyl]-4-(4-N,N-dipropylphenyl) imidazole,
2-[4-(3-ethoxy-trans-1-propen-1-yl)phenyl]-4-(4-N-isopropylphenyl) imidazole,
2-[4-(3-ethoxy-trans-1-propen-1-yl)phenyl]-4-(4-N-isobutylphenyl) imidazole,
2-[4-(3-ethoxy-trans-1-propen-1-yl)phenyl]-4-(4-N-morpholinophenyl) imidazole,
2-[4-(3-ethoxy-trans-1-propen-1-yl)phenyl]-4-[4-N-(N'-ethyl)-piperizanophenyl) imidazole,
2-[4-(3-ethoxy-trans-1-propen-1-yl)phenyl]-4-(4-N-morpholinophenyl)-5-methylimidazole.

Preferred compositions of the invention include compositions comprising compounds as defined above in structural formula (1) (or pharmaceutically acceptable salts, prodrugs, esters, or solvates of these compounds) in admixture with a pharmaceutically acceptable diluent, adjuvent, or carrier.

Provided according to the invention, therefore, are novel compounds which modulate multi-drug resistance (MDR) in vitro in CEM/VLB1000 human cells.

Provided according to the invention, therefore, are novel compounds which modulate multi-drug resistance (MDR) in murine models with P388-ADR human cells.

Provided according to the invention, therefore, are novel compounds which modulate multi-drug resistance (MDR) in murine models with P388-ADR ascites human tumors.

Another aspect of the present invention provides compositions comprising MDR modulating compounds of the invention suitable for administration to a mammalian host.

As a preferred embodiment, the compounds of the invention may be used as therapeutics to modulate MDR in cancer patients who show resistance to anticancer chemotherapeutic agents such as DOX, taxol and VLB.

Preferred embodiments of the invention further include use of compounds of the invention in pharmaceutical preparations to increase the sensitization of MDR cancer cells in patients who show resistance to anticancer chemotherapeutic agents such as DOX, taxol and VLB.

Compounds of the invention may additionally be used for the manufacture of medicaments for the treatment or modulation of MDR in animals, including commercially important animals.

Provided according to this invention are medicaments for sensitizing multi-drug resistant tumor cells comprising the novel compounds of the present invention.

Provided according to this invention are see page 3 treatment of MDR or drug-sensitive tumor cells to be administered prior to, together with, or subsequent to the administration of an antitumor chemotherapeutic agent.

Provided according to this invention are pharmaceutical compositions for increasing the sensitivity of tumor cells to antitumor chemotherapeutic agents and thus for the treatment of tumors that are susceptible to anti-cancer chemotherapeutic agents but have become resistant to such chemotherapy.

The compounds of Formula (1) maybe prepared by procedures known to those skilled in the art from known compounds or readily preparable intermediates.

The following Examples are intended to illustrate the preparation of compounds of Formula 1, and as such are not intended to limit the invention as set forth in the claims appended thereto. Furthermore, the compounds described in the following examples are not to be construed as forming the only genus that is considered as the invention, and any combination of the compounds or their moieties may itself form a genus. The structure and purity of all final products were assured by at least one of the following methods: thin -layer chromatography (TLC), mass spectroscopy, nuclear magnetic resonance (NMR) spectroscopy. NMR data is in the form of delta (d) values for major diagnostic protons, given in parts per million (ppm) relative to tetramethylsilane (TMS) as internal standard, determined at 400 MHz in deuterated sovents such as deuteriochloroform (CDCl₃), and deuteriomethanol (CD₃OD); conventional abbreviations used for signal shape are: s, singlet; d, doublet; t, triplet; dd, double of doublet; dt, double of triplet; m, multiplet; br., broad; etc. The following abbreviations have also been used: mL (milliliter), g (gram), mg (milligram), mol (moles), mmol (millimoles), equiv (equivalent).

The procedures employed to synthesize compounds depicted in Formula 1 are as follows:

### Method A. General Procedure for the Preparation of Diones:

There are three methods by which these diones were synthesized, namely:

### Method 1

4,4'-difluorodione 4 was reacted with a series of amines (R₁R₂NH) using an appropriate base such as K₂CO₃, Na₂CO₃, Et₃N, diisopropylethylamine (DIEA), etc., at elevated temperature (60-150°C) in an appropriate solvent such as alcohol, acetonitrile, N,N-dimethylformamide (DMF), dimethylsulfoxide (DMSO) to provide the mono-amino-diones 2 (procedure Bader et al J. Org. Chem. 1966, 31, 2319). The mono-amino-diones 2 were further reacted with another amine (R₃R₄NH) under the same conditions to afford the desired diones 3 as shown in Scheme 1. This procedure allows for the synthesis of unsymmetrical diones 6 (wherein R₁R₂NH is different from R₃R₄NH). This chemistry was carried out using 1-1.5 equivalent of R₁R₂NH and upon the completion of the reaction another equivalent of different amine (R₃R₄NH) was added to the reaction mixture to provide the desired unsymmetrical diones (Scheme 1).

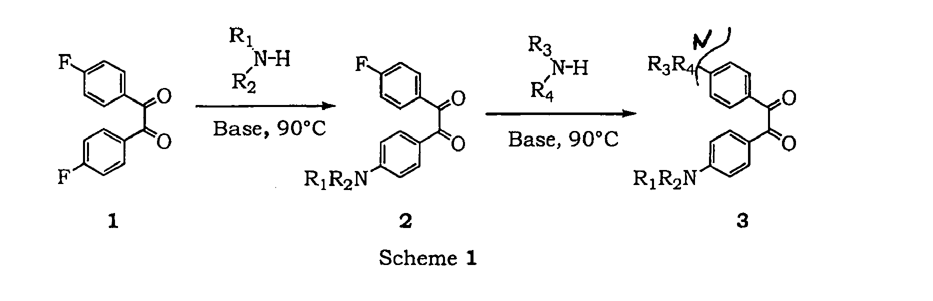

Unsymmetrcal diones were prepared according to the following procedure: To a solution of 4,4'-difluorobenzil in DMSO (0.5 M) was added 1.2 equiv of amine R₁R₂NH and 2 equiv of potassium carbonate. The resulting mixture was stirred in a 90°C oil bath for 6-15 hours (TLC monitoring). After completion, the mixture was diluted with ether and extracted with 3 M hydrochloric acid (x5) to remove the small amount of product resulted from the di-displacement. The organic layer was then washed with 6 M hydrochloric acid until no more desired product could be detected in the ether layer (5 times). The aqueous layer was neutralized to pH 8 with 6 M aqueous sodium hydroxide and it was extracted with dichloromethane. The organic layers were dried (Na₂SO₄), evaporated to give 4-amino,4'-fluorobenzil. This procedure was repeated with the second amine R₃R₄NH (normally 2-3 equiv) and a simple workup by diluting the reaction mixture into ether and washed with water to remove DMSO. 4, 4'-diaminobenzil was thus obtained (50-90% overall depending on the amines used) in high purity.

For symmetrical diones (wherein R₁R₂NH is equal to R₂R₃NH) the following procedure was followed:
To a solution of 4,4'-difluorobenzil in DMSO (0.5 M) was added 2-3 equiv of amine R₁R₂NH and 2-3 equiv of potassium carbonate. The resulting mixture was stirred in an 90°C oil bath for 6-15 hours (TLC monitoring). After completion, the mixture was diluted into ether and washed with water to remove DMSO. The desired diones 6 were obtained (50-90% overall depending amines used) in high purity. The following examples have been synthesized according to method **1**:

### Examples

### 4) 4-N,N-dimethylamino-4'-methylaminobenzil

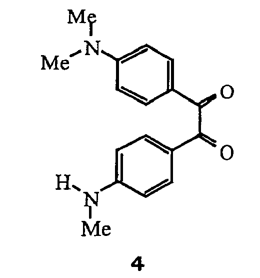

¹H NMR (400 MHz, CDCl₃) δ 2.80 (s, 3H), 3.03 (s, 6H), 4.48 (br s, 1H), 6.48 (d, 2H), 6.59 (d, 2H), 7.75 (d, 2H), 7.79 (d, 2H).

### 5) 4-N,N-diethylamino-4'-methylaminobenzil

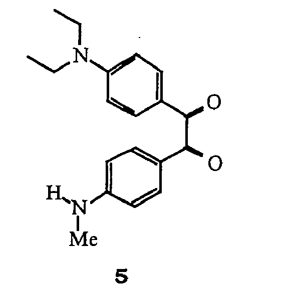

¹H NMR (400 MHz, CDCl₃) δ 1.15 (t, 6H), 2.85 (s, 3H), 3.37 (q, 4H), 4.40 (s, 1H), 6.50 (d, 2H), 6.57 (d, 2H), 7.78 (d, 4H).

### 6) 4-N-isopropylamino-4'-methylaminobenzil

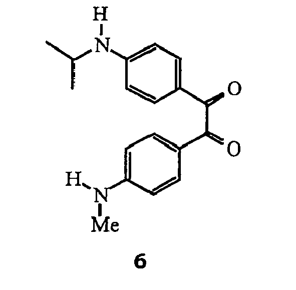

¹H NMR (400 MHz, CDCl₃) δ 1.18 (d, 6H), 2.83 (d, 3H), 3.65 (m, 1H), 4.28 (br d, 1H), 4.54 (br s, 1H), 6.47 (d, 2H), 6.49 (d, 2H), 7.74 (d, 2H), 7.76 (d, 2H).

### 7) 4-pyrrolidino-4'-methylaminobenzil

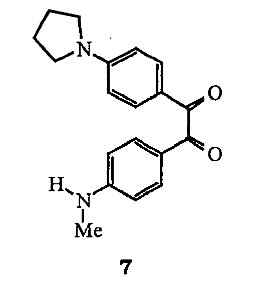

¹H NMR (400 MHz, CDCl₃) δ 1.98 (m, 4H), 2.83 (s, 3H), 3.32 (m, 4H), 4.48 (s, 1H), 6.46 (d, 2H), 6.48 (d, 2H), 7.76 (d, 2H), 7.78 (d, 2H).

### 8) 4-piperidino-4'-methylaminobenzil

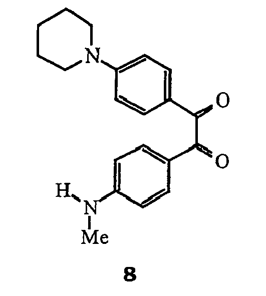

¹H NMR (400 MHz, CDCl₃) δ 1.61 (br s, 6H), 2.83 (d, 3H), 3.35 (br s, 4H), 4.48 (s, 1H), 6.49 (d, 2H), 6.77 (d, 2H), 7.76 (d, 2H), 7.78 (d, 2H).

### 9)4-N,N-dimethylamino-4'-N-(2-methoxyethyl)aminobenzil

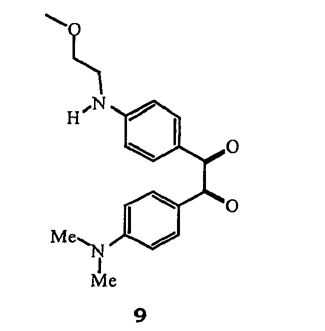

¹H NMR (400 MHz, CDCl₃) δ 3.03 (s, 6H), 3.32 (m, 5H), 3.56 (t, 2H), 4.66 (s, 1H), 6.53 (d, 2H), 6.60 (d, 2H), 7.77 (d, 2H), 7.80 (d, 2H).

### 10)4-N,N-di-(2-methoxyethyl)amino-4'-methylaminobenzil

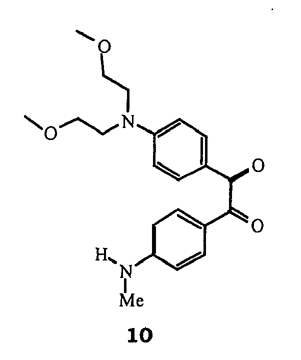

¹H NMR (400 MHz, CDCl₃) δ 2.82 (d, 3H), 3.29 (s, 6H), 3.50 (t, 4H), 3.60 (t, 4H), 4.48 (br s, 1H), 6.49 (d, 2H), 6.64 (d, 2H), 7.76 (d, 4H).

### 11)4-(imidazol-1-yl)-4'-N-(2-methoxyethyl)aminobenzil

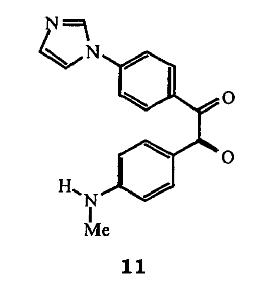

¹H NMR (400 MHz, CD₃OD) δ 2.82 (s, 3H), 6.59 (d, 2H), 7.15 (s, 1H), 7.69 (m, 3H), 7.76 (d, 2H), 8.05 (d, 2H), 8.29 (s, 1H).

### 12)4,4'-bis(4-morpholino)benzil

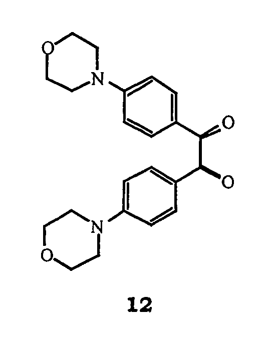

¹H NMR (400 MHz, CDCl₃) δ 3.30 (m, 8H), 3.80 (m, 8H), 6.80 (d, 4H), 7.82 (d, 4H).

### 13)4-N,N-dimethylamino-4'-(4-morpholino)benzil

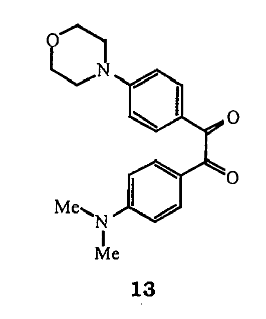

¹H NMR (400 MHz, CDCl₃) δ 3.04 (s, 6H), 3.30 (m, 4H), 3.81 (m, 4H), 6.61 (d, 2H), 6.82 (d, 2H), 7.81 (d, 2H), 7.85 (d, 2H).

### 14)4-N-methylamino-4'-(4-morpholino)benzil

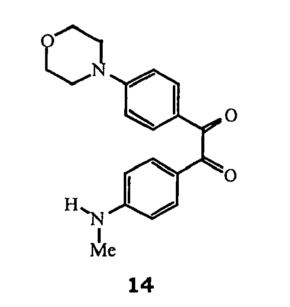

¹H NMR (400 MHz, CDCl₃) δ 2.90 (d, 3H), 3.30 (m, 4H), 3.80 (m, 4H), 4.42 (m, 1H), 6.52 (d, 2H), 6.82 (d, 2H), 7.78 (d, 2H), 7.84 (d, 2H).

### 15)4-N,N-diallylamino-4'-fluorobenzil

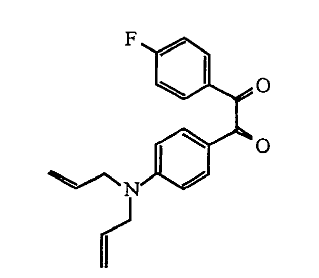

¹H NMR (400 MHz, CDCl₃) δ 3.95 (d, 4H), 5.12 (m, 4H), 5.78 (m, 2H), 6.63 (d, 2H), 7.09 (d, 1H), 7.10 (d, 1H), 7.75 (d, 2H), 7.96 (m, 2H).

### Method 2

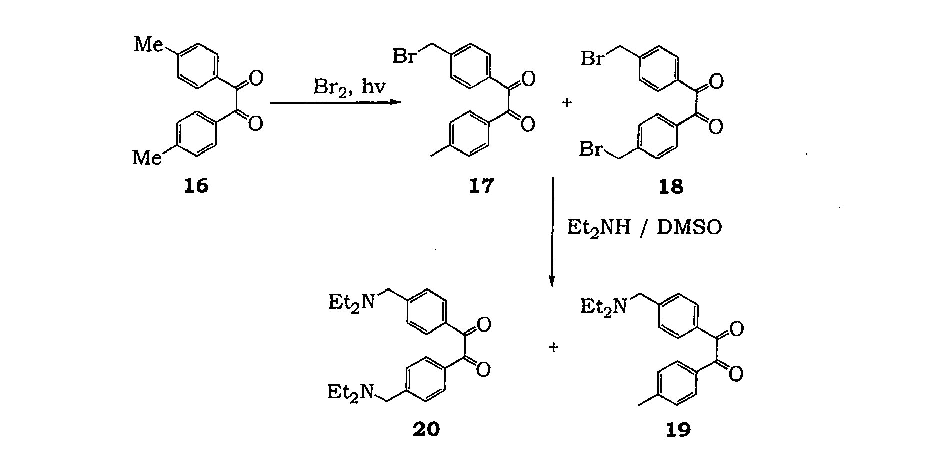

Compound **19** and **20** were prepared according to Venugopalan et al (Indian J. Chem. 1991, 30B, 777-783).

Compound **19** has: ¹H NMR (400 MHz, CDCl₃) δ 1.00 (t, 6H), 2.39 (s, 3H), 2.50 (q, 4H), 3.60 (s, 3H), 3.61 (s, 2H), 7.26 (d, 2H), 7.45 (d, 2H), 7.83 (d, 2H), 7.86 (d, 2H).

Compound **20** has: ¹H NMR (400 MHz, CDCl₃) δ 1.00 (t, 12H), 2.50 (q, 8H), 3.60 (s, 4H), 7.46 (d, 4H), 7.87 (d, 4H).

### Method B. General method for the synthesis of aldehydes

### Method B-1

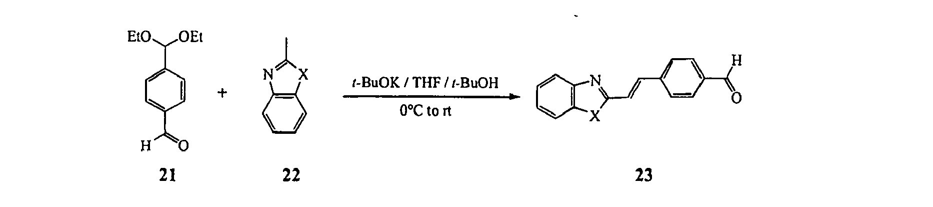

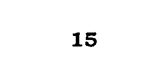

Aldehydes 23 were prepared according to Houpis et al (J. Org. Chem. 1993, 58, 3176-3178).

### Examples

### 25)p-[trans-2-(benzoxazol-2-yl)ethenyl]benzaldehyde

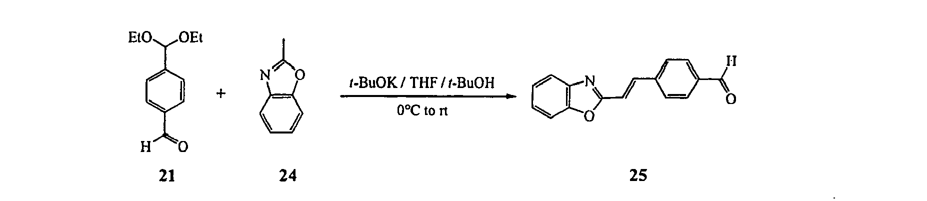

To a solution of terephthaldehyde mono (diethyl acetal) **21** (5.000 g, 24 mmol.), 2-methylbenzoxazole in 5:1 THF-*t*-BuOH (77.4 mL) cooled at -5°C, was added *t*-BuOK in THF (1.0 M, 36.0 mL, 36.0 mmol.) in such a rate to keep the internal temperature of the reaction below 0°C (ca. 10 min). The resulting mixture was stirred under nitrogen overnight during which time the temperature rised up to room tempt. It was then diluted with ethyl acetate and washed with sat. sodium bicarbonate. The organic layer was dried (Na₂SO₄) and evaporated to give a brown oily solid. It was then dissolved in boiling methanol (50 mL) and cooled to room tempt. The white solid was precipitated out after the addition of water (25 mL) and the solid was collected. The acetal thus obtained was hydrolysed by stirring the product in 3:1 THF-1 N HCl solution for 10 min. The mixture was extracted with ethyl acetate, the organic layers were washed with sat. sodium bicarbonate, brine and dried (Na₂SO₄). Evaporation gave a slightly yellow solid **25**, 4.43 g (74% overall yield). Compound **25** has: ¹H NMR (400 MHz, CDCl₃) δ 7.14 (d, 1H), 7.31 (m, 2H), 7.49 (m, 1H), 7.68 (m, 3H), 7.75 (d, 1H), 7.86 (d, 2H), 10.00 (s, 1H).

### 26)p-[trans-2-(benzthiazol-2-yl)ethenyl]benzaldehyde

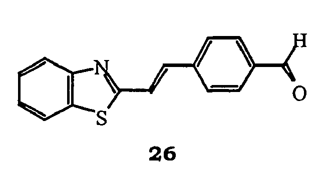

Compound **26** has: ¹H NMR (400 MHz, CDCl₃) δ 7.36 (dd, 1H), 7.44 (dd, 1H), 7.50 (d, 2H), 7.68 (d,, 2H), 7.84 (d, 1H), 7.88 (d, 2H), 7.98 (d, 1H), 9.98 (s, 1H).

### Method B-2

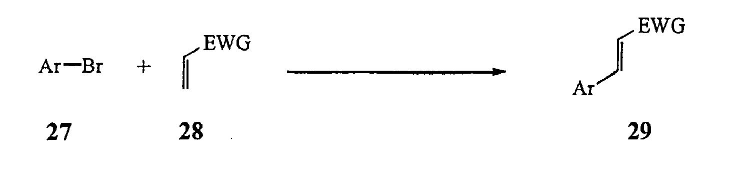

By allowing a compound (**27**) wherein Ar is defined as above to react with compound (**28**) wherein EWG is esters and other electron withdrawing groups, under the following conditions, the desired compounds (**29**) can be obtained.

These reactions may be carried out neat or in a solvent such as dimethylformamide (DMF), tetrahydrofuran (THF), and toluene in the presence of a catalyst (e.g. Pd(OAc)₂, Pd(PPh₃)₄, Pd₂dba₃), a ligand (e.g. Ph₃P, Ph₃As, (o-tolyl)₃P) and a base (e.g. K₂CO₃, CsCO₃, Et₃N) at temperatures ranging from 23°C to 130°C, for 1 to 60 hours.

### Examples

### 32)Methyl 4-formyl trans-cinnamate

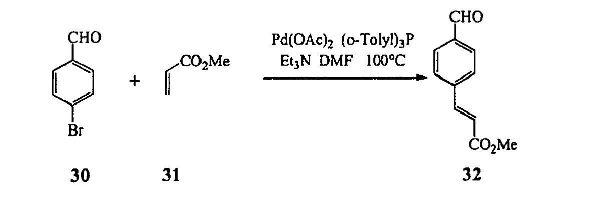

Prepared according to Patel et al (J. Org. Chem., 1977, 42, 3903).

Compound **32** has: ¹H NMR (400 MHz, CDCl₃) δ 3.78 (s, 3H), 6.50 (d, 1H), 7.63 (m, 3H), 7.85 (d, 2H), 9.98 (s, 1H)..

### 33)p-[trans-2-(methylsulfonyl)ethenyl]benzaldehyde

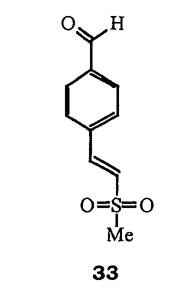

Compound **33** has: ¹H NMR (400 MHz, CDCl₃) δ 3.00 (s, 3H), 7.01 (d, 1H), 7.63 (d, 1H), 7.64 (d, 2H), 7.90 (d, 2H).

### Method B-3

### 36)p-[trans-2-(cyano)ethenyl)benzaldehyde

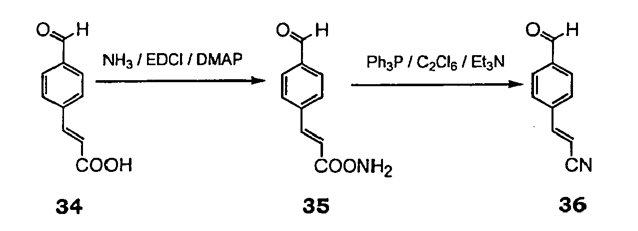

Amonia (0.5 M in dioxane, 45 mL, 22.5 mmol.) was added to a suspension of **34** 2.10 g, 12 mmol.), EDCI (2.37 g, 14.4 mmol.) and DMAP (0.293 g, 2.4 mmol.) in dichloromethane (50 mL). The resulting mixture was stirred at room tempt overnight. It was then diluted with dichloromethane and washed with 1.0 M hydrochloric acid, followed by sat. sodium bicarbonate. The organic layer was dried (Na₂SO₄) and evaporated to give 1.244 g of compound **35** as a sightly yellow solid.

To a solution of Compound **35** (340 mg, 1.94 mmol.) in dichloromethane (20.0 mL) was added triethylamine (1.62 mL, 11.64 mmol.), thriphenylphosphine (1.5 g, 5.8 mmol.) and hexachloroethane (1.37 g, 5.8 mmol.). The resulting mixture was stirred at room temperature for 10 min and evaporated. Flash chromatography of the residue (silica, 2.0 x 10 cm) by using 20 and 30% ethyl acetate - hexanes gave compound **36** , 118 mg. Compound **36** has: ¹H NMR (400 MHz, CDCl₃) δ 5.98 (d, 1H), 7.57 (d, 2H), 7.61 (d, 1H), 7.87 (d, 2H), 1.00 (s, 1H).

### Method B-4

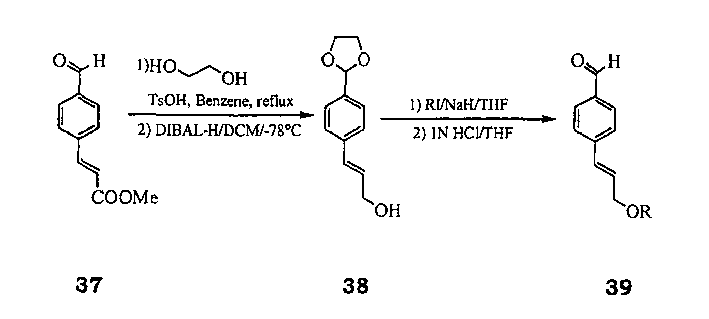

A solution of aldehyde 37 (2.3 g, 12.1 mmol), ethylene glycol (1.35 mL, 24.2 mmol) , and p-toluenesulfonic acid (10 mg, catalytic amount) in benzene (30.0 mL) was refluxed for 2 h. Then it was diluted with ethyl acetate and washed with sat. aqueous sodium bicarbonate and brine, dried (Na₂SO₄), evaporated. The crude material thus obtained was dissolved in dichloromethane (DCM, 100.0 mL) and cooled to -78°C. DIBAL-H (1.0 M in DCM, 45 mL, 45.mmol) was added over 20 min. Aqueous NaOH (1.0 M, 100 mL) was added and the mixture was warmed to room temperature (23°C) and the layers were separated. The aqueous layer was extracted with DCM (X3), and the combined organic layers were dried (Na₂SO₄) and evaporated. Flash chromatography of the residue over silica gel gave the desired allylic alcohol **37**. Compound 37 has: ¹H NMR (400 MHz, CDCl₃) d 4.00 (d, 2H), 4.10 (m, 2H), 4.30 (d, 2H), 6.35 (dt, 1H), 6.60 (d, 1H), 7.48 (m, 4H).

Alkylation of allylic alcohol **38** with alkyl iodide and sodium hydride in THF following the standard procedure (Jung, M. E. et al., Tetrahedron Lett., 1989, 30, 641) and hydrolysis of the resulting acetal with 1 N aqueous HCl gave the corresponding allylic ether **39**.

### Examples

### 40)p-(3-methoxy-trans-1-propen-1-yl) benzaldehyde

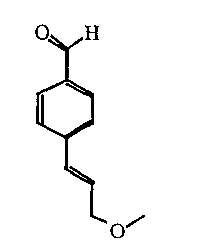

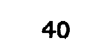

Compound **40** has: ¹H NMR (400 MHz, CDCl₃) δ 3.20 (s, 3H), 4.10 (d, 2H), 6.40 (dt, 1H), 6.64 (d, 1H), 7.49 (d, 2H), 7.80 (d, 2H).

### 41)p-(3-ethoxy-trans-1-propen-1-yl) benzaldehyde

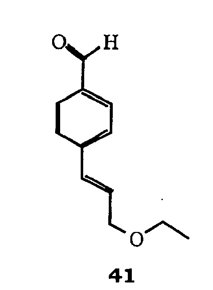

Compound **41** has: ¹H NMR (400 MHz, CDCl₃) δ 1.23 (t, 3H), 3.54 (q, 2H), 4.14 (d, 2H), 6.42 (dt, 1H), 6.64 (d, 1H), 7.49 (d, 2H), 7.79 (d, 2H).

### 42)p-(3-benzyloxy-trans-1-propen-1-yl) benzaldehyde

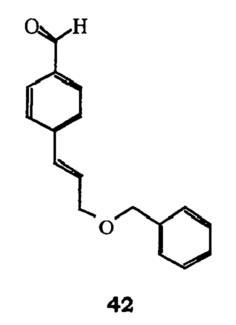

Compound **42** has: ¹H NMR (400 MHz, CDCl₃) δ 4.20 (d, 2H), 4.56 (s, 2H), 6.46 (dt, 1H), 6.67 (d, 1H), 7.34 (m, 5H), 7.49 (d, 2H), 7.79 (d, 2H).

### 43)p-(3-butyloxy-trans-1-propen-1-yl) benzaldehyde

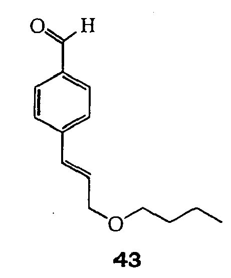

Compound **43** has: ¹H NMR (400 MHz, CDCl₃) δ 0.90 (t, 3H), 1.39 (m, 2H), 1.57 (m, 2H), 3.47 (t, 2H), 4.13 (d, 2H), 6.43 (m, 1H), 6.64 (d, 1H), 7.49 (d, 2H), 7.79 (d, 2H), 9.94 (s, 1H).

### 44)p-[3-(2-methoxyethyl)-trans-1-propen-1-yl)]benzaldehyde

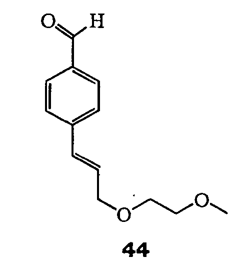

Compound **44** has: ¹H NMR (400 MHz, CDCl₃) δ 3.38 (s, 3H), 3.55 (m, 2H), 3.64 (m, 2H), 4.20 (d, 2H), 6.43 (m, 1H), 6.64 (d, 1H), 7.48 (d, 2H), 7.79 (d, 2H), 9.94 (s, 1H).

### Method B-5

### 46)p-(3-phenoxy-trans-1-propen-1-yl) benzaldehyde

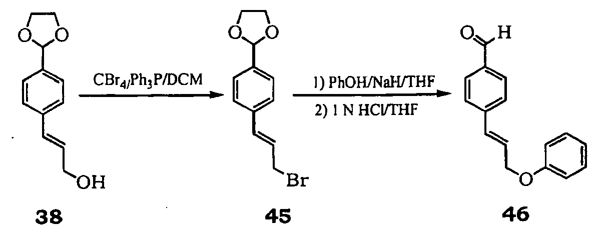

Carbon tetrabromide (723 mg, 2.18 mmol) was added, in one prtion, to a solution of allylic alcohol **38** (300 mg, 1.45 mmol), and triphenylphosphine (456 mg, 1.75 mmol) in DCM at room temperature (23°C). After 2 min, sat aqueous sodium bicarbonate was added and the layers were separated. The aqueous layer was extracted with DCM once and the combined organic layers were dried (Na₂SO₄), and evaporated. Flash chromatography of the residue over silica gel gave a white solid 344 mg (88%). Allylic bromide **45** has: ¹H NMR (400 MHz, CDCl₃) d 4.20 (d, 2H), 4.56 (s, 2H), 6.46 (dt, 1H), 6.67 (d, 1H), 7.34 (m, 5H), 7.49 (d, 2H), 7.79 (d, 2H).

The mixture of allylic bromide **45** (50 mg, 0.185 mmol), phenol (35 mg, 0.37 mmol), and sodium hydride (excess) in THF (2.0 mL) was heated at 50 °C for 5 h. 1 N aqueous HCl was added after cooling down to 23°C, 20 min later, the mixture ws diluted with ethyl acetate and washed with 1 N NaOH. The organic layer was dried (Na₂SO₄), and evaporated. Purification of the residue on preparative TLC gave the desired aldehyde **46**, 24 mg, as a white solid. Compound **46** has: ¹H NMR (400 MHz, CDCl₃) δ 4.70 (d, 2H), 6.55 (dt, 1H), 6.77 (d, 1H), 6.94 (m, 3H), 7.28 (m, 2H), 7.51 (d, 2H), 7.81 (d, 2H), 9.98 (s, 1H).

### 47)p-[3-(3,4-dimethoxyphenoxy)-trans-1-propen-1-yl) benzaldehyde

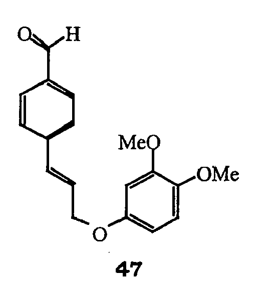

Compound **47** has: ¹H NMR (400 MHz, CDCl₃) δ 3.80 (s, 3H), 3.82 (s, 3H), 4.63 (d, 2H), 6.44 (m, 1H), 6.54 (m, 2H), 6.75 (m, 2H), 7.50 (d, 2H), 7.79 (d, 2H), 9.96 (s, 1H).

### Method B-6

### 48)p-[3-(1-morpholino)-trans-1-propen-1-yl] benzaldehyde

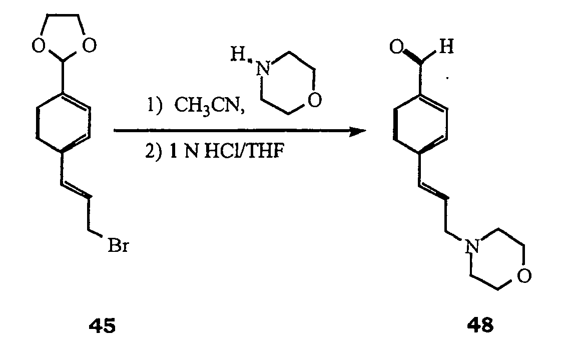

Morpholine (82 mL, 0.945 mmol) was added to a solution of allylic bromide **45** in acetonitrile (3.0 mL). 30 min later, 1 N aqueous HCl was added and the resulting mixture was stirred for 20 min. It was then diluted with ethyl acetate and washed with sat. aqueous Na₂CO₃, dried (Na₂SO₄). Evaporation off the solvents gave the desired product **48**, 55 mg. Compound **48** has: ¹H NMR (400 MHz, CDCl₃) δ 2.50 (m, 4H), 3.20 (d, 2H), 3.64 (m, 4H), 6.46 (m, 1H), 6.65 (d, 1H), 7.58 (d, 2H), 7.82(d, 2H), 9.92 (s, 1H).

### 49)p-[3-(1-piperidino)-trans-1-propen-1-yl) benzaldehyde

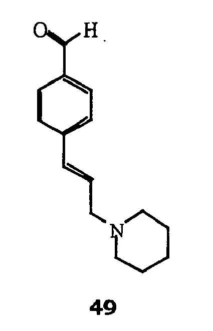

Compound **49** has: ¹H NMR (400 MHz, CDCl₃) δ 1.24 (m, 2H), 1.60 (m, 4H), 2.50 (m, 4H), 3.18 (d, 2H), 6.46 (m, 1H), 6.64 (d, 1H), 7.58 (d, 2H), 7.82(d, 2H), 9.92 (s, 1H).

### 50)p-(3-N,N-dimethylamino-trans-1-propen-1-yl) benzaldehyde

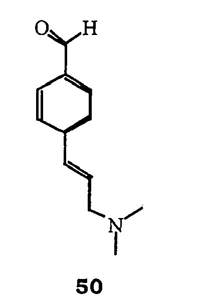

Compound **50** has: ¹H NMR (400 MHz, CDCl₃) δ 2.30 (s, 6H), 3.18 (d, 2H), 6.46 (m, 1H), 6.64 (d, 1H), 7.58 (d, 2H), 7.82(d, 2H), 9.92 (s, 1H).

### Method B-7

### 51)p-(3-N,N-diethylcarbamate-trans-1-propen-1-yl) benzaldehyde

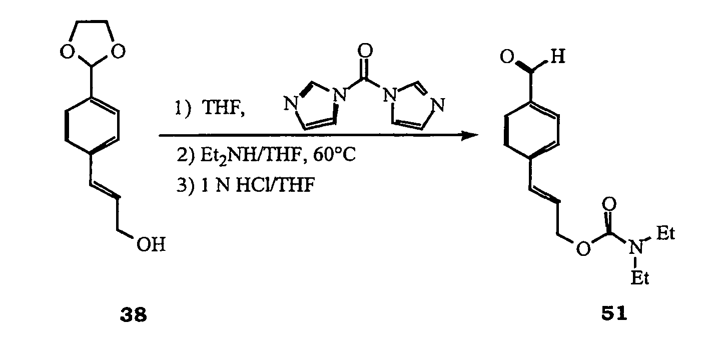

Carbonyl diimidazole (66 mg, 0.407 mmol) was added to a solution of allylic alcohol **38** (42 mg, 0.204 mmol) in THF. The resulting mixture was stirred for 1 h at 23°C and diethylamine (63 mL, 0.612 mmol). It was then heated up to 60 °C for overnight. 1 N HCl was then added and the resulting mixture was stirred for 20 min. It was then diluted with ethyl acetate and washed with water. The organic layer was dried and evaporated. The crude material (30 mg) was chromatographically pure. Compound **51** has: ¹H NMR (400 MHz, CDCl₃) 8 1.10 (t, 6H), 3.28 (m, 4H), 4.75 (d, 2H), 6.44 (m, 1H), 6.62 (d, 1H), 7.50 (d, 2H), 7.80 (d, 2H), 9.95 (s, 1H).

### Method B-8

### 52)p-[trans-(2-methoxycarbonylcyclopropan- 1-yljbenzaldehyde

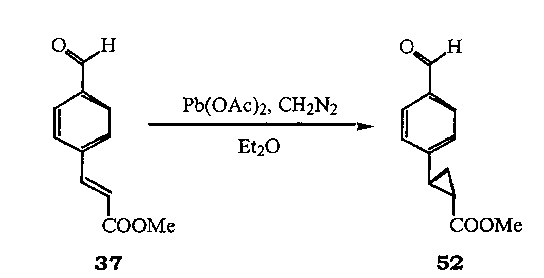

Diazomethane (0.3 M in Et₂O, 8.6 mL, 2.6 mmol) was added to a suspension of compound **37** (123 mg, 0.64 mmol), palladium acetate (catalytic amount) in ether (1.0 mL). After stirring at 23°C overnight, it was quenched with acetic acid. The mixture was diluted with DCM, washed with sat. aqueous sodium carbonate, and dried (Na₂SO₄). Evaporation off the solvents gave the desired compound as an oil (99 mg). Compound **52** has: ¹H NMR (400 MHz, CDCl₃) δ 1.35 (m, 1H), 1.60 (m, 1H), 1.92 (m, 1H), 2.50 (m, 1H), 3.64 (s, 3H), 7.18 (d, 2H), 7.78 (d, 2H), 9.90 (s, 1H).

### Method C. General Procedure for the Preparation of Imidazoles:

### Method 1

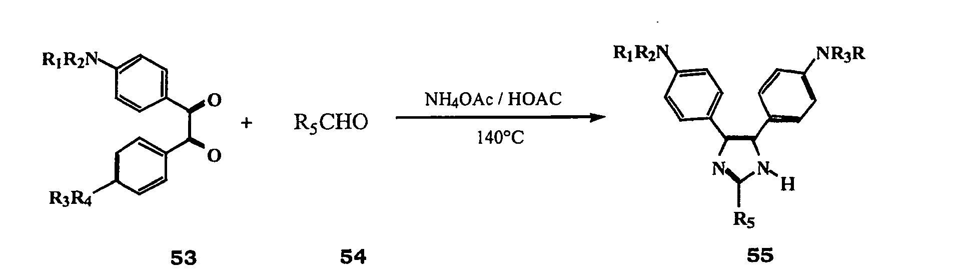

Imidazoles **55** were synthesed according to modified literature procedure (Krieg et al Z Naturforsch teil 1967, 22b, 132).

The proper dione (3.04 mmol.) and aldehyde (4.56 mmol.) were placed in acetic acid (5.85 mL) and ammonium acetate (30.4 mmol.) was placed in acetic acid (1.75 mL) in a separated reaction flask. Both of the flasks were heated in an preheated oil bath (140°C). As soon as the solids in the two flasks were dissolved, poured the hot solution of ammonium acetate in acetic acid into the other flask which contains the aldehyde and dione. The resulting mixture was heated at 140°C for 40 min. It was then cooled to room temperature. The pH of solution was adjusted to 0.8 using 3.0 M hydrochloric acid. It was then extracted with ether (5 times) to remove the unreacted aldehyde and dione). The aqueous layer was neutralized to pH 8 with 3 M sodium hydroxide and extracted with methylenechloride (3 times). The organic layers were dired (N₂SO₄) and evaporated to give the corresponding imidazole compound.

### Example 56

2-[trans-2-(2-benzoxazolyl)ethenylphenyl]-4,5-bis (4-N,N-dimethylaminophenyl) imidazole: 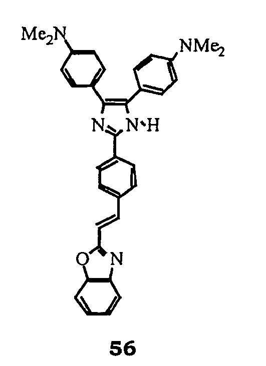 ¹H NMR (400 MHz, CDCl₃) δ 2.95 (s, 12H), 6.55 (d, 4H), 6.90 (d, 1H), 7.21 (m, 6H), 7.39 (m, 1H), 7.50 (m, 3H), 7.63 (d, 1H), 7.83 (d, 2H); ESIMS, m/z for C₃₄H₃₁ON₅ [M+H]⁺: 526.

### Example 57

2-[trans-2-(2-benzoxazolyl)ethenylphenyl]-4-(4-N,N-dimethylaminophenyl)-5-(4-N-methylaminophenyl) imidazole: 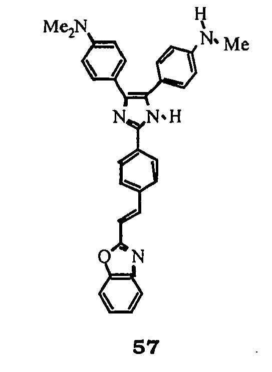 ¹H NMR (400 MHz, CD₃OD) δ 2.78 (s, 3H), 2.95 (s, 6H), 6.51 (d, 2H), 6.63 (d, 2H), 6.98 (d, 2H), 7.36 (m, 8H), 7.64 (m, 1H), 7.70 (d, 1H), 7.85 (d, 2H); ESIMS, m/z for C₃₃H₂₉ON₅ [M+H]⁺: 512.

### Example 58

2-[trans-2-(2-benzoxazolyl)ethenylphenyl]-4-(4-N,N-diethylaminophenyl)-5-(4-N-methylaminophenyl) imidazole: 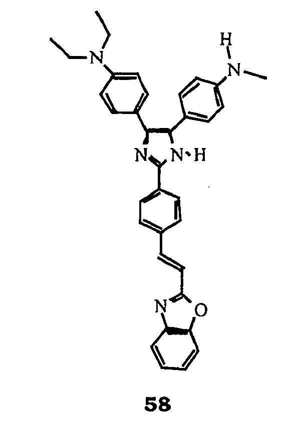 ¹H NMR (400 MHz, CDCl₃) δ 1.12 (t, 6H), 2.80 (s, 3H), 3.31 (q, 4H), 6.53 (d, 2H), 6.59 (d, 2H), 6.98 (d, 1H), 7.29 (m, 2H), 7.39 (m, 4H), 7.49 (m, 1H), 7.52 (d, 2H), 7.65 (m, 1H), 7.70 (d, 1H), 7.86 (d, 2H); ESIMS, m/z for C₃₅H₃₃ON₅ [M+H]⁺: 540.

### Example 59

2-[trans-2-(2-benzoxazolyl)ethenylphenyll-4-(4-N-disopropylaminophenyl)-5-(4-N-methylaminophenyl) imidazole: 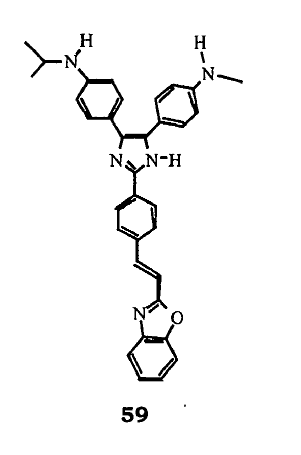 ¹H NMR (400 MHz, CDCl₃) δ 1.14 (s, 3H), 1.16 (s, 3H), 2.76 (s, 3H), 3.56 (m, 1H), 6.48 (m, 4H), 6.92 (d, 1H), 7.31 (m, 6H), 7.46 (m, 3H), 7.62 (m, 1H), 7.66 (d, 1H), 7.83 (d, 2H); ESIMS, m/z for C₃₄H₃₁ON₅ [M+H]⁺: 526.

### Example 60

2-[trans-2-(2-benzoxazolyl)ethenylphenyl)-4-(4-N-methylaminophenyl)-5-(4-pyrrolidinophenyl) imidazole: 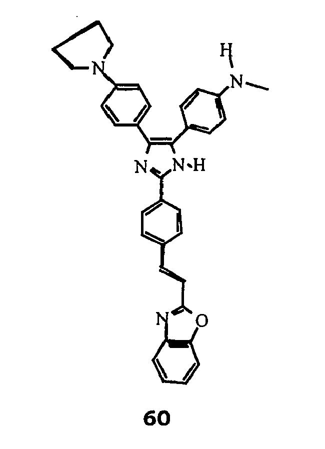 ¹H NMR (400 MHz, CDCl₃) δ 1.95 (br s, 4H), 2.80 (br s, 3H), 3.30 (br s, 4H), 6.50 (m, 4H), 7.00 (br d, 1H), 7.22-7.90 (m, 13H); ESIMS, m/z for C₃₅H₃₁ON₅ [M+H]+: 538.

### Example 61

2-[trans-2-(2-benzoxazolyl)ethenylphenyl]-4-(4-N,N-dimethylaminophenyl)-5-[4-(2-methoxyethylamino)phenyl] imidazole: 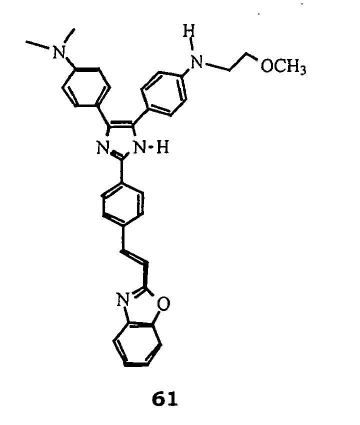 ¹H NMR (400 MHz, CDCl₃) δ 2.90 (s, 6H), 3.24 (br s, 2H), 3.35 (s, 3H), 3.56 (t, 2H), 6.54 (d, 2H), 6.63 (d, 2H), 6.97 (d, 1H), 7.24-7.44 (m, 6H), 7.49 (m, 3H), 7.64 (m, 1H), 7.69 (s, 1H), 7.85 (d, 2H); ESIMS, m/z for C₃₅H₃₃O₂N₅ [M+H]⁺: 556.

### Example 62

2-[trans-2-(2-benzthiazolyl)ethenylphenyl]-4,5-bis (4-N,N-dimethylaminophenyl) imidazole: 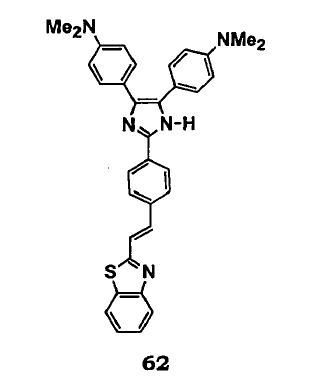 ¹H NMR (400 MHz, CDCl₃) δ 2.85 (s, 12H), 6.62 (d, 4H), 7.26-7.56 (m, 9H), 7.76-7.88 (m, 3H), 7.92 (d, 2H); ESIMS, m/z for C₃₄H₃₁SN₅ [M+H]⁺: 542.

### Example 63

2-[trans-2-(2-benzthiazolyl)ethenylphenyl]-4-(4-N,N-dimethylaminophenyl)-5-(4-N-methylaminophenyl) imidazole: 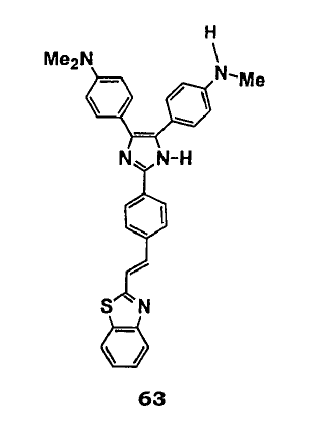 ¹H NMR (400 MHz, CDCl₃) δ 2.80 (s, 3H), 2.90 (s, 6H), 6.53 (d, 2H), 6.64 (d, 4H), 7.26-7.50 (m, 6H), 7..54 (d, 2 H), 7.80 (d, 1H), 7.85 (d, 2H), 7.93 (d, 1H); ESIMS, m/z for C₃₃H₂₉SN₅ [M+H]⁺: 528.

### Example 64

2-[trans-2-(2-cyano)ethenylphenyl]-4,5-(4-N,N-dimethylaminophenyl) imidazole: 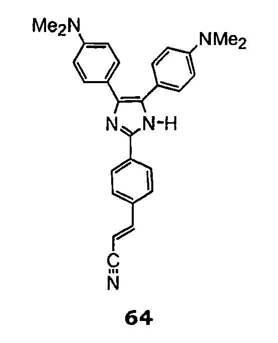 ¹H NMR (400 MHz, CDCl₃) δ 2.95 (s, 12H), 5.80 (d, 1H), 6.65 (d, 4H), 7.40 (m, 7H), 7.85 (br s, 2H); ESIMS, m/z for C₂₈H₂₇N₅ [M+H]⁺: 434.

### Example 65

2-[trans-2-(2-cyano)ethenylphenyl)-4-(4-N,N-dimethylaminophenyl)-5-[4-N-(2-methoxyethyl)amino)phenyl] imidazole: 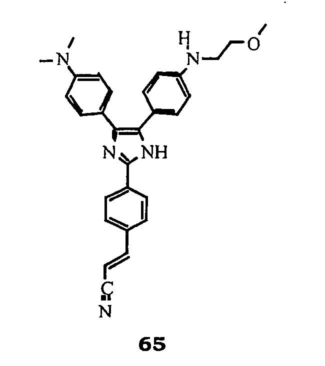 ¹H NMR (400 MHz, CD₃OD) δ 2.92 (s, 6H), 3.25 (m, 2H), 3.34 (s, 3H), 3.54 (t, 2H), 6.20 (d, 1H), 6.60 (d, 2H), 6.70 (d, 2H), 7.26 (m, 4H), 7.50 (d, 1H), 7.60 (d, 2H), 7.96 (d, 2H); ESIMS, m/z for C₂₉H₂₉N₅O[M+H]⁺: 464.

### Example 66

2-(trans-2-methoxycarbonyl-ethenylphenyl)-4-(4-N,N-diallylaminophenyl)-5-(4-fluoro-phenyl) imidazole: 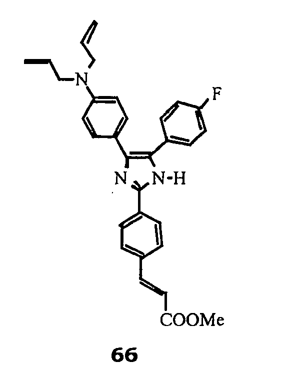 Compound 66 was prepared according the method C by using the proper dione and aldehyde. Compound 66 has: ¹H NMR (400 MHz, CDCl₃) δ 3.76 (s, 3H), 3.91 (s, 4H), 5.16 (m, 4H), 5.83 (m, 2H), 6.41 (d, 1H), 6.63 (d, 2H), 6.96 (m, 2H), 7.24 (d, 2H), 7.57 (m, 5H), 7.84 (d, 2H); ESIMS, m/z for C₃₁H₂₈O₂N₃F [M+H]⁺: 494.

### Example 67

2-(trans-2-methoxycarbonyl-ethenylphenyl)-4-(4-N-methylaminophenyl)-5-(4-pyrrolidinophenyl) imidazole: 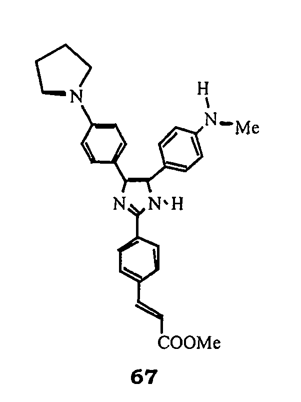 Compound **67** was prepared according the method C by using the proper dione and aldehyde. Compound **67** has: ¹H NMR (400 MHz, CD₃OD) δ 1.96 (m, 4H), 2.74 (s, 3H), 3.10 (s, 4H), 3.78 (s, 3H), 6.42-6.56 (m, 5H), 7.24 (dd, 4H), 7.58 (d, 2H), 7.64 (d, 1H), 7.91 (d, 2H); ESIMS, m/z for C₃₀H₃₀O₂N₄ [M+H]⁺: 479.

### Example 68

2-(trans-2-methoxycarbonyl-ethenylphenyl)-4-(4-N-methylaminophenyl)-5-(4-piperidinophenyl) imidazole: 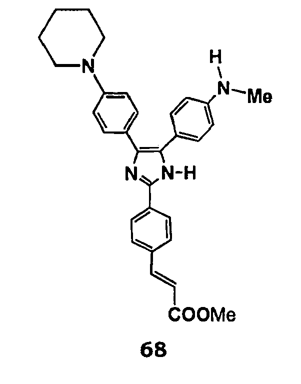 Compound **68** was prepared according the method C by using the proper dione and aldehyde. Compound **68** has: ¹H NMR (400 MHz, CD₃OD) δ 1.54 (m, 2H), 1.64 (m, 4H), 2.74 (s, 3H), 3.08 (s, 4H), 3.78 (s, 3H), 6.48 (d, 1H), 6.54 (d, 2H), 6.85 (d, 2H), 7.20 (d, 2H), 7.31 (d, 2H), 7.58 (d, 2H), 7.63 (d, 1H), 7.91 (d, 2H); ESIMS, m/z for C₃₁H₃₂O₂N₄ [M+H]⁺: 493.

### Example 69

2-(trans-2-methoxycarbonyl-ethenylphenyl)-4-[4-N,N-di(2-methoxyethyl)aminophenyl]-5-(4-N-methylaminophenyl) imidazole: 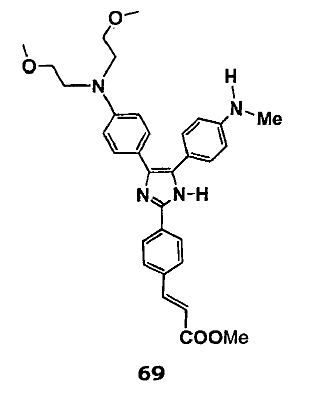 ¹H NMR (400 MHz, CDCl₃) δ 2.82 (s, 3H), 3.30 (s, 6H), 3.52 (m, 8H), 3.80 (s, 3H), 6.42 (d, 1H), 6.60 (m, 4H), 7.30-7.60 (m, 6H), 7.68 (d, 1H), 7.90 (br s, 2H); ESIMS, *m*/*z* for C₃₂H₃₆O₄N₄ [M+H]⁺: 541.

### Example 70

2-(trans-2-methoxycarbonyl-ethenylphenyl)-4-[4-(1-imidazolyl)phenyl]-5-(4-N-methylaminophenyl) imidazole: 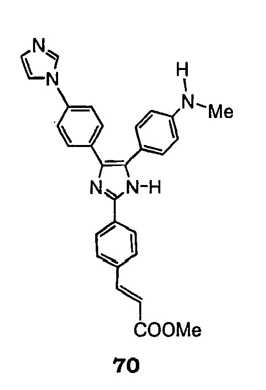 ¹H NMR (400 MHz, CDCl₃) δ 2.80 (s, 3H), 3.72 (s, 3H), 6.36 (d, 1H), 6.53 (d, 2H), 7.06 (s, 1H), 7.21 (d, 4H), 7.48 (d, 2H), 7.59 (d, 1H), 7.62 (d, 2H), 7.74 (s, 1H), 7.89 (d, 2H); ESIMS, m/z for C₂₉H₂₅O₂N₅ [M+H]⁺: 476.

### Example 71

2-(trans-2-methoxycarbonyl-ethenylphenyl)-4,5-bis (4-N-morpholinophenyl) imidazole: 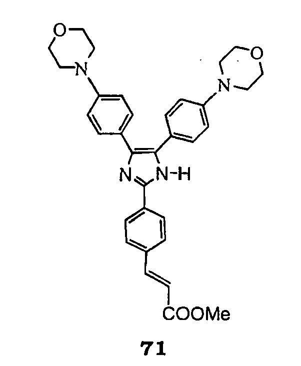 ¹H NMR (400 MHz, CDCl₃) δ 3.10 (t, 8H), 3.74 (s, 3H), 3.79 (t, 8H), 6.36 (d, 1H), 6.79 (d, 4H), 7.36 (d, 4H), 7.47 (d, 2H), 7.59 (d, 1H), 7.86 (d, 2H); ESIMS, m/z for C₃₃H₃₄O₄N₄ [M+H]⁺: 551.

### Example 72

2-(trans-2-methoxycarbonyl-ethenylphenyl)-4-(4-N,N-dimethylaminophenyl)-5-(4-N-morpholinophenyl) imidazole: 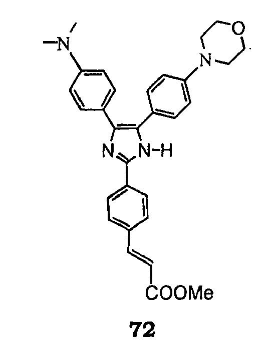 ¹H NMR (400 MHz, CDCl₃) δ 2.90 (br s, 6H), 3.10 (br s, 4H), 3.76 (s, 3H), 3.82 (m, 4H), 6.40 (d, 1H), 6.65 (d, 2H), 6.81 (d, 2H), 7.28-7.56 (m, 6H), 7.63 (d, 1H), 7.84 (d, 2H); ESIMS, m/z for C₃₁H₃₂O₃N₄ [M+H]⁺: 509.

### Example 73

2-(trans-2-methoxycarbonyl-ethenylphenyl)-4-(4-N-methylaminophenyl)-5-(4-N-morpholinophenyl) imidazole: 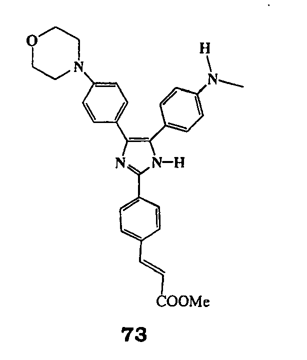 ¹H NMR (400 MHz, CDCl₃) δ 2.80 (br s, 3H), 3.10 (m, 4H), 3.76 (s, 3H), 3.82 (t, 4H), 6.37 (d, 1H), 6.52 (d, 2H), 6.80 (d, 2H), 7.28-7.56 (m, 6H), 7.61 (d, 1H), 7.83 (d, 2H); ESIMS, m/z for C₃₀H₃₀O₃N₄ [M+H]⁺: 495.

### Example 74

2-[4-(3-methoxy-trans-1-propen-1-yl)phenyl]-4,5-bis (4-N,N-dimethylaminophenyl) imidazole: 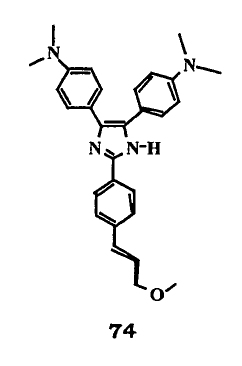 ¹H NMR (400 MHz, CD₃OD) δ 2.90 (s, 12H), 3.35 (s, 3H), 4.10 (d, 2H), 6.35 (m, 1H), 6.65 (d, 1H), 6.70 (d, 4H), 7.30 (d, 4H), 7.50 (d, 2H), 7.90 (d, 2H); ESIMS, m/z for C₂₉H₃₂ON₄ [M+H]⁺: 453.

### Example 75

2-[4-(3-ethoxy-trans-1-propen-1-yl)phenyl]-4,5-bis (4-N,N-dimethylaminophenyl) imidazole: 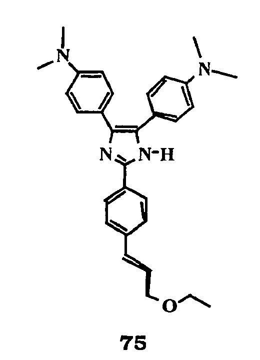 ¹H NMR (400 MHz, CD₃OD) δ 1.10 (t, 3H), 2.90 (s, 12H), 3.50 (q, 2H), 4.10 (br s, 2H), 6.35 (m, 1H), 6.65 (d, 1H), 6.70 (d, 4H), 7.30 (br s, 4H), 7.50 (d, 2H), 7.90 (br s, 2H); ESIMS, m/z for C₃₀H₃₄ON₄ [M+H]+: 467.

### Example 76

2-[4-(3-ethoxy-trans-1-propen-1-yl)phenyl]-4-(4-N,N-dimethylaminophenyl)-5-(4-N-methylaminophenyl) imidazole: 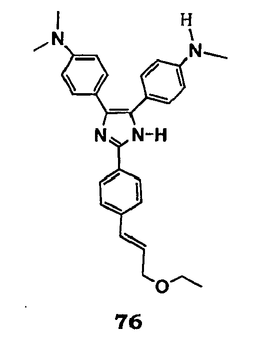 ¹H NMR (400 MHz, CD₃OD) δ 1.36 (t, 3H), 2.90 (s, 3H), 3.08 (s, 6H), 3.70 (q, 2H), 4.28 (d, 2H), 6.45 (m, 1H), 6.71 (d, 2H), 6.76 (d, 1H), 6.85 (d, 2H), 7.39 (d, 2H), 7.47 (d, 2H), 7.60 (d, 2H), 8.02 (d, 2H); ESIMS, m/z for C₂₉H₃₂ON₄ [M+H]⁺: 453.

### Example 77

2-[4-(3-benzyloxy-trans-1-propen-1-yl)phenyl]-4,5-bis (4-N,N-dimethylaminophenyl) imidazole: 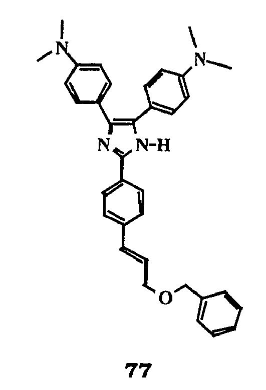 ¹H NMR (400 MHz, CD₃OD) δ 2.90 (s, 12H), 4.17 (d, 2H), 4.54 (s, 2H), 6.38 (m, 1H), 6.64 (d, 1H), 6.69 (d, 4H), 7.30 (m, 9H), 7.46 (d, 2H), 7.86 (d, 2H); ESIMS, m/z for C₃₅H₃₆ON₄ [M+H]⁺: 529.

### Example 78

2-[4-(3-phenoxy-trans-1-propen-1-yl)phenyl]-4-(4-N,N-dimethylaminophenyl)-5-(4-N-methylaminopheny) imidazole: 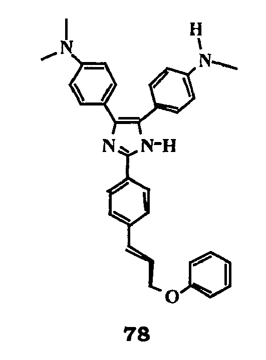 ¹H NMR (400 MHz, CD₃OD) δ 2.70 (s, 3H), 2.90 (s, 6H), 4.68 (d, 2H), 6.48 (m, 1H), 6.57 (d, 1H), 6.72 (m, 4H), 6.88 (t, 1H), 6.94 (d, 1H), 7.24 (m, 4H), 7.32 (d, 2H), 7.48 (d, 2H), 7.88 (d, 2H).

### Example 79

2-{4-[3-(3,4-dimethoxy-phenoxy)-trans-1-propen-1-yl]phenyl}-4-(4-N,N-dimethylaminophenyl)-5-(4-N-methylaminopheny) imidazole: 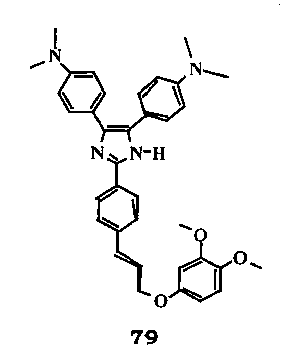 ¹H NMR (400 MHz, CD₃OD) δ 2.90 (s, 12H), 3.70 (s, 3H), 3.78 (s, 3H), 5.10 (m, 2H), 6.30 (m, 1H), 6.44 (s, 1H), 6.70 (m, 6H), 7.30 (m, 7H), 7.80 (d, 2H).

### Example 80

2-[4-(3-N,N-diethylamino-trans-1-propen-1-yl)phenyl]-4,5-bis (4-N,N-dimethylaminophenyl) imidazole: 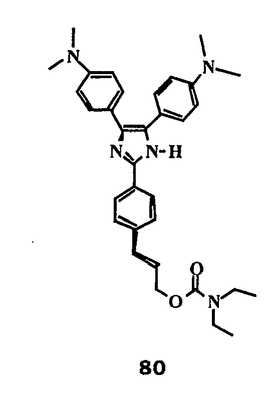 ¹H NMR (400 MHz, CD₃OD) δ 1.10 (m, 6H), 2.90 (s, 12H), 3.28 (m, 4H), 4.70 (d, 2H), 6.34 (m, 1H), 6.65 (d, 1H), 6.67 (d, 4H), 7.30 (d, 4H), 7.46 (d, 2H), 7.86 (d, 2H).

### Example 81

2-[4-(3-N-morpholino-trans-1-propen-1-yl)phenyl]-4,5-bis (4-N,N-dimethylaminophenyl) imidazole: 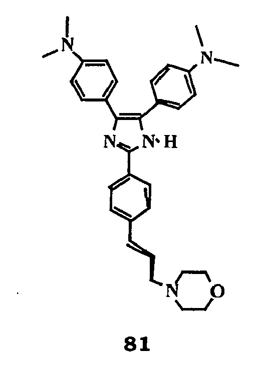 ¹H NMR (400 MHz, CD₃OD) δ 2.49 (br s, 4H), 2.89 (s, 12H), 3.15 (d, 2H), 3.67 (dd, 4H), 6.29 (m, 1H), 6.58 (d, 1H), 6.69 (d, 4H), 7.29 (d, 4H), 7.44 (d, 2H), 7.86 (d, 2H); ESIMS, m/z for C₃₂H₃₇ON₅ [M+H]⁺: 508.

### Example 82

2-[4-(3-N-piperidino-trans-1-propen-1-yl)phenyl]-4,5-bis (4-N,N-dimethylaminophenyl) imidazole: 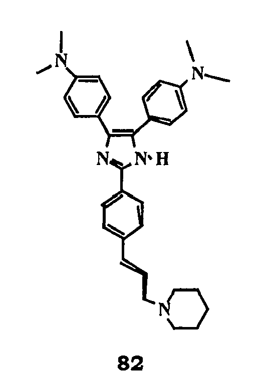 ¹H NMR (400 MHz, CD₃OD) δ 1.45 (br s, 2H), 1.60 (m, 4H), 2.47 (br s, 4H), 2.89 (s, 12H), 3.13 (d, 2H), 6.30 (m, 1H), 6.55 (d, 1H), 6.69 (d, 4H), 7.29 (d, 4H), 7.43 (d, 2H), 7.86 (d, 2H); ESIMS, m/z for C₃₃H₃₉N₅ [M+H]⁺: 506.

### Example 83

2-[4-(3-N,N-dimethylamino-trans-1-propen-1-yl)phenyl]-4,5-bis (4-N,N-dimethylaminophenyl) imidazole: 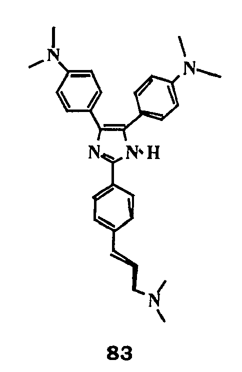 ¹H NMR (400 MHz, CD₃OD) δ 2.28 (s, 6H), 2.89 (s, 12H), 3.13 (d, 2H), 6.28 (m, 1H), 6.56 (d, 1H), 6.67 (d, 4H), 7.29 (d, 4H), 7.43 (d, 2H), 7.86 (d, 2H); ESIMS, m/z for C₃₀H₃₅N₅ [M+H]⁺: 466.

### Example 84

2-{4-[3-(2-methoxy-ethoxy)-trans-1-propen-1-yl]phenyl}-4,5-bis (4-N,N-dimethylaminophenyl) imidazole: 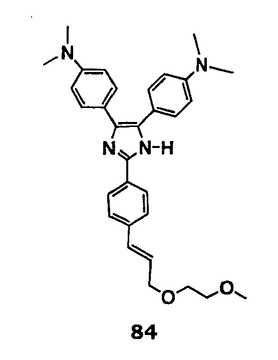 ¹H NMR (400 MHz, CD₃OD) δ 2.90 (s, 12H), 3.34 (s, 3H), 3.55 (m, 2H), 3.62 (m, 2H), 4.16 (d, 2H), 6.36 (m, 1H), 6.64 (d, 1H), 6.70 (d, 4H), 7.30 (d, 4H), 7.46 (d, 2H), 7.87 (d, 2H); ESIMS, m/z for C₃₁H₃₆O₂N₄ [M+H]⁺: 497.

### Example 85

2-[4-(3-butoxy-trans-1-propen-1-yl)phenyl]-4,5-bis (4-N,N-dimethylaminophenyl) imidazole: 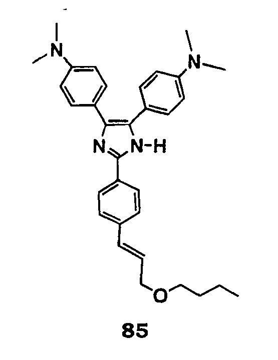 ¹H NMR (400 MHz, CD₃OD) δ 0.91 (t, 3H), 1.39 (m, 2H), 1.56 (m, 2H), 2.09 (s, 12H), 3.47 (t, 2H), 4.10 (d, 2H), 6.34 (m,. 1H), 6.61 (d, 2H), 6.69 (d, 4H), 7.29 (d, 4H), 7.44 (d, 2H), 7.86 (d, 2H); ESIMS, m/z for C₃₂H₃₈ON₄ [M+H]⁺: 495.

### Example 86

2-[4-(3-ethoxy-trans-1-propen-1-yl)phenyl]-4,5-bis (4-N,N-diethylaminophenyl) imidazole: 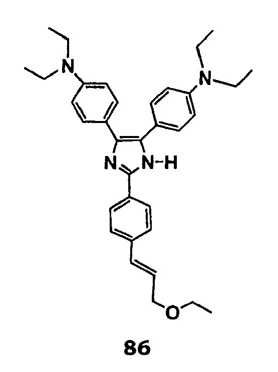 ¹H NMR (400 MHz, CD₃OD) δ 1.10 (t, 12H), 1.20 (t, 3H), 3.30 (br s, 8H), 3.55 (q, 2H), 4.08 (d, 2H), 6.34 (m, 1H), 6.58 (m, 5H), 7.20 (d, 4H), 7.40 (d, 2H), 7.80(d, 2H); ESIMS, m/z for C₃₄H₄₂ON₄ [M+H]⁺: 523.

### Example 87

2-[4-(3-ethoxy-trans-1-propen-1-yl)phenyl]-4-(4-N,N-diethylaminophenyl)-5-(4-N-methylaminophenyl) imidazole: 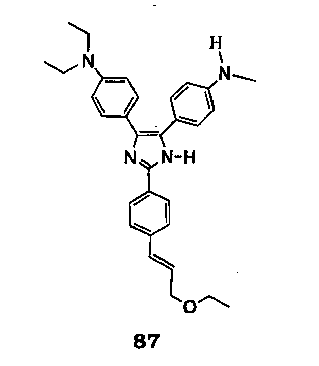 ¹H NMR (400 MHz, CD₃OD) δ 1.10 (t, 6H), 1.19 (t, 3H), 2.74 (s, 3H), 3.33 (q, 4H), 3.52 (q, 2H), 4.10 (d, 2H), 6.34 (m, 1H), 6.59 (m, 5H), 7.25 (m, 4H), 7.44(d, 2H), 7.85 (d, 2H); ESIMS, m/z for C₃₁H₃₆ON₄ [M+H]⁺: 481.

### Example 88

2-[4-(3-methoxy-trans-1-propen-1-yl)phenyl]-4,5-bis (4-pyrrolidinophenyl) imidazole: 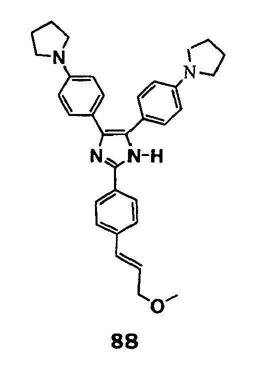 ¹H NMR (400 MHz, CD₃OD) δ 1.97 (m, 8H), 3.28 (m, 8H), 3.36 (s, 3H), 4.12 (d, 2H), 6.35 (m, 1H), 6.50 (d, 4H), 6.62 (d, 1H), 7.27 (d, 4H), 7.46 (d, 2H), 7.87 (d, 2H); ESIMS, m/z for C₃₃H₃₆ON₄ [M+H]⁺: 505.

### Example 89

2-[4-(3-ethoxy-trans-1-propen-1-yl)phenyl]-4,5-bis (4-pyrrolidinophenyl) imidazole: 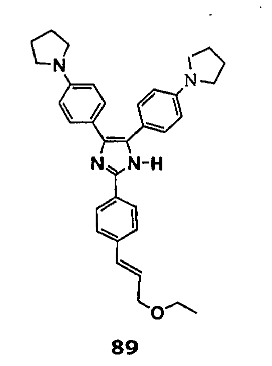 ¹H NMR (400 MHz, CD₃OD) δ 1.19 (t, 3H), 1.97 (m, 8H), 3.28 (m, 8H), 3.53 (q, 2H), 4.12 (d, 2H), 6.35 (m, 1H), 6.50 (d, 4H), 6.62 (d, 1H), 7.27 (d, 4H), 7.46 (d, 2H), 7.87 (d, 2H); ESIMS, m/z for C₃₄H₃₈ON₄ [M+H]⁺: 519.

### Example 90

2-[4-(3-ethoxy-trans-1-propen-1-yl)phenyl]-4-(4-N,N-dimethylaminophenyl)-5-(4-pyrrolidinophenyl) imidazole: 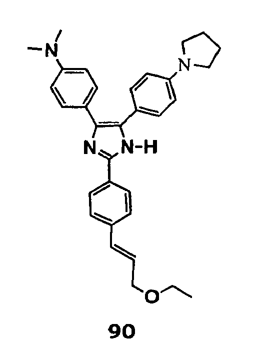 ¹H NMR (400 MHz, CD₃OD) δ 1.17 (t, 3H), 1.91 (m, 4H), 2.85 (s, 6H), 3.16 (br s, 4H), 3.50 (q, 2H), 4.07 (d, 2H), 6.30 (m, 1H), 6.43 (d, 2H), 6.57 (d, 1H), 6.63 (d, 2H), 7.21 (d, 2H), 7.27 (d, 2H), 7.40 (d, 2H), 7.82 (d, 2H); ESIMS, m/z for C₃₂H₃₆ON₄ [M+H]⁺: 493.

### Example 91

2-[4-(3-ethoxy-trans-1-propen-1-yl)phenyl]-4-(4-N-methylaminophenyl)-5-(4-pyrrolidinophenyl) imidazole: 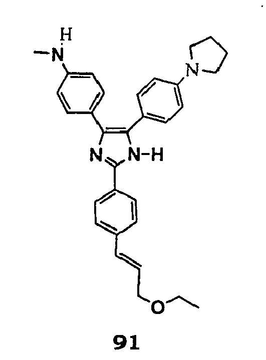 ¹H NMR (400 MHz, CD₃OD) δ 1.17 (t, 3H), 1.91 (m, 4H), 2.85 (s, 3H), 3.16 (br s, 4H), 3.50 (q, 2H), 4.07 (d, 2H), 6.30 (m, 1H), 6.43 (d, 2H), 6.57 (d, 1H), 6.63 (d, 2H), 7.21 (d, 2H), 7.27 (d, 2H), 7.40 (d, 2H), 7.82 (d, 2H); ESIMS, m/z for C₃₁H₃₄ON₄ [M+H]⁺: 479.

### Example 92

2-[4-(3-ethoxy-trans-1-propen-1-yl)phenyl]-4,5-bis (4-N-morpholinophenyl) imidazole: 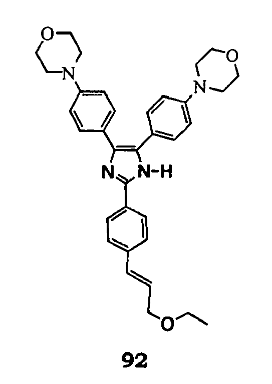 ¹H NMR (400 MHz, CD₃OD) δ 1.18 (t, 3H), 3.10 (br s, 8H), 3.56 (m, 2H), 3.78 (br s, 8H), 4.14 (d, 2H), 6.38 (m, 1H), 6.88 (d, 4H), 7.36 (d, 4H), 7.48 (d, 2H), 7.88 (d, 2H); ESIMS, m/z for C₃₄H₃₈O₃N₄ [M+H]⁺: 551.

### Example 93

2-[4-(3-ethoxy-trans-1-propen-1-yl)phenyl]-4-(4-N,N-dimethylaminophenyl)-5-(4-N-morpholinophenyl) imidazole: 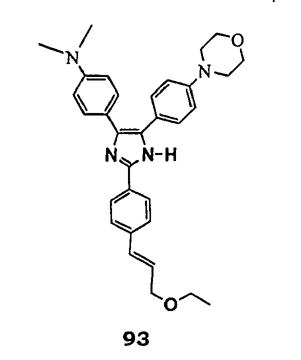 ¹H NMR (400 MHz, CD₃OD) δ 1.18 (t, 3H), 2.90 (br s, 6H), 3.09 (m, 4H), 3.52 (q, 2H), 3.76 (m, 4H), 4.12 (d, 2H), 6.34 (m, 1H), 6.62 (d, 1H), 6.69 (d, 2H), 6.86 (d, 2H), 7.27 (d, 2H), 7.34 (d, 2H), 7.45 (d, 2H), 7.87 (d, 2H); ESIMS, m/z for C₃₂H₃₆O₂N₄ [M+H]⁺: 509.

### Example 94

2-[4-(3-ethoxy-trans-1-propen-1-yl)phenyl]-4-(4-N-methylaminophenyl)-5-(4-N-morpholinophenyl) imidazole: 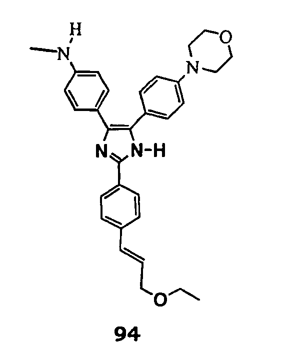 ¹H NMR (400 MHz, CD₃OD) δ 1.19 (t, 3H), 2.73 (s, 3H), 3.08 (m, 4H), 3.52 (q, 2H), 3.76 (m, 4H), 4.10 (d, 2H), 6.34 (m, 1H), 6.55 (d, 2H), 6.61 (d, 1H), 6.85 (d, 2H), 7.20 (d, 2H), 7.34 (d, 2H), 7.45 (d, 2H), 7.86 (d, 2H); ESIMS, m/z for C₃₁H₃₄O₂N₄ [M+H)⁺: 495.

### Example 95

2-[4-(3-ethoxy-trans-1-propen-1-yl)phenyl]-4-(4-N-methylaminophenyl)-5-(4-N-isopropylaminophenyl) imidazole: 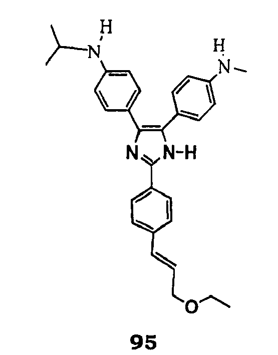 ¹H NMR (400 MHz, CD₃OD) δ 1.15 (s, 3H), 1.16 (s, 3H), 1.18 (t, 3H), 2.74 (s, 3H), 3.53 (m, 3H), 4.10 (d, 2H), 6.33 (m, 1H), 6.56 (m, 4H), 6.60 (d, 1H), 7.23 (t, 4H), 7.44 (d, 2H), 7.85 (d, 2H); ESIMS, m/z for C₃₀H₃₄ON₄ [M+H]⁺: 467.

### Example 96

2-[4-trans-(2-methanesulfonyl-ethenyl)-phenyl]-4,5-bis (4-N,N-dimethylaminophenyl) imidazole: 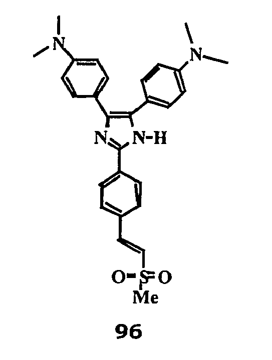 ¹H NMR (400 MHz, CD₃OD) δ 2.90 (s, 12H), 3.04 (s, 3H), 6.70 (d, 4H), 7.28 (d, 1H), 7..32 (d, 4H), 7.58 (d, 1H), 7.68 (d, 2H), 8.00 (d, 2H); ESIMS, m/z for C₂₈H₃₀O₂N₄S [M+H]⁺: 487.

### Example 97

2-(4-N-morpholinophenyl)-4,5-bis (4-N,N-dimethylaminophenyl) imidazole: 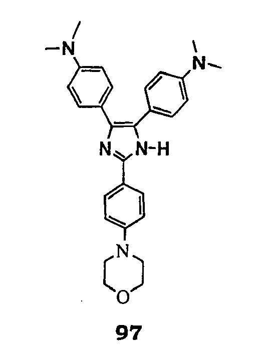 ¹H NMR (400 MHz, CD₃OD) δ 2.85 (br s, 12H), 3.10 (t, 4H), 3.74 (t, 4H), 6.64 (d, 4H), 6.92 (d, 2H), 7.26 (d, 4H), 7.76 (d, 2H); ESIMS, m/z for C₂₉H₃₃ON₅ [M+H]⁺: 468.

### Example 98

2-[4-(5-ethylcarboxyisoxazol-3-yl)-phenyl]-4,5-bis (4-N,N-dimethylaminophenyl) imidazole: 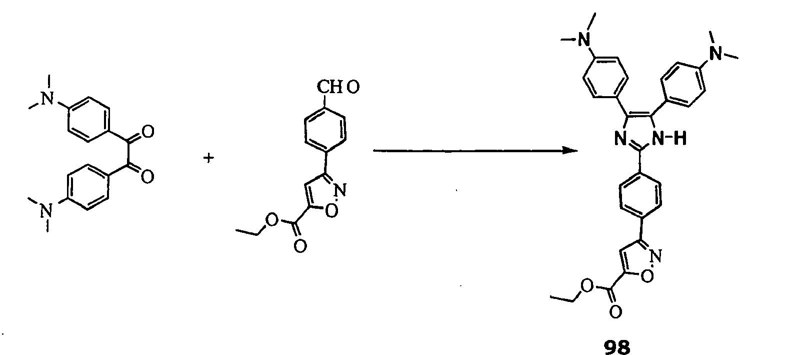 The aldehyde was prepared from terephthaldehyde mono (diethyl acetal) according to a similar literature preparation (*Cf.* Moriya, O. et al. J. Chem. Soc. Perkin Trans 1, 1994, 413.).
Imidazole **98**was prepared according to method C-1. Compound **96** has: ¹H NMR (400 MHz, CD₃OD) δ 1.20 (t, 3H), 2.90 (br s, 12H), 4.40 (q, 2H), 6.90 (br s, 4H), 7.30 ( br s, 2H), 7.58 (s, 1H), 7.90 (br s, 4H), 8.30 (br s, 2H); ESIMS, m/z for C₃₁H₃₁O₃N₅ [M+H]⁺: 522.

### Example 99

2-[4-trans-(2-methoxycarbonyl-ethenyl)phenyl]-4-(p-tolyl)-5-(4-N,N-diethylaminomethylphenyl) imidazole: 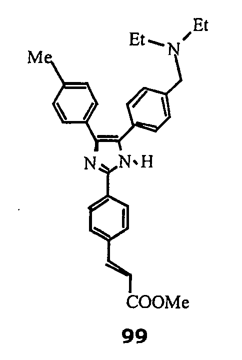 ¹H NMR (400 MHz, CDCl₃) δ 1.01 (t, 6H), 2.30 (s, 3H), 2.51 (q, 4H), 3.54 (s, 2H), 3.76 (s, 3H), 6.38 (d, 1H), 7.04-7.54 (m, 10H), 7.62 (d, 1H), 7.92 (d, 2H); ESIMS, *m*/*z* for C₃₁H₃₃O₂N₃ [M+H]⁺: 480.

### Example 100

2-[4-trans-(2-methoxycarbonyl-ethenyl)phenyl]-4,5-bis (4-N,N-diethylaminomethylphenyl) imidazole: 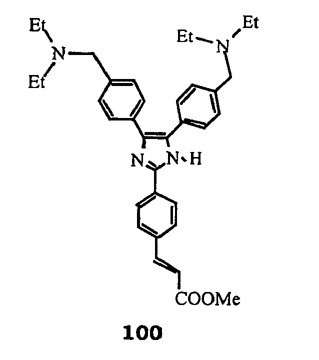 ¹H NMR (400 MHz, CDCl₃) δ 1.01 (t, 12H), 2.51 (q, 8H), 3.54 (s, 4H), 3.76 (s, 3H), 6.40 (d, 1H), 7.20-7.54 (m, 10H), 7.64 (d, 1H), 7.92 (d, 2H); ESIMS, *m*/*z* for C₃₅H₄₂O₂N₄ [M+H]⁺: 551.

### Example 101

2-[4-trans-(2-methoxycarbonyl)cyclopropan-1-yl]-4,5-bis (4-N,N-diethylaminomethylphenyl) imidazole: 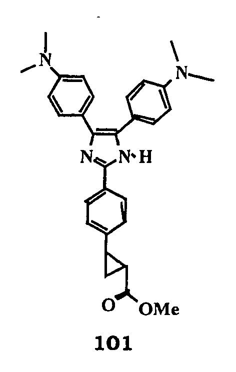 ¹H NMR (400 MHz, CD₃OD) δ 1.38 (m, 1H), 1.53 (m, 1H), 1.92 (m, 1H), 2.48 (m, 1H), 2.90 (s, 12 H), 3.68 (s, 3H), 6.69 (d, 4H), 7.17 (d, 2H), 7.28 (d, 4H), 7.82 (d, 2H); ESIMS, *m*/*z* for C₃₀H₃₂O₂N₄ [M+H]⁺: 481.

### Example 102

2-[4-(3-ethoxy-trans-1-propen-1-yl)phenyl]-4,5-bis (4-dimethoxyphenyl) imidazole: 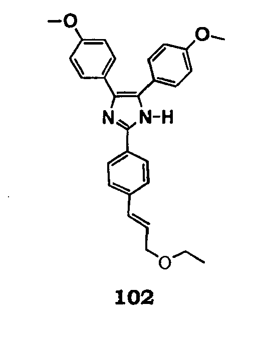 ¹H NMR (400 MHz, CD₃OD) δ 1.18 (t, 3H), 3.50 (q, 2H), 3.72 (s, 6H), 4.08 (d, 2H), 6.33 (m, 1H), 6.58 (d, 1H), 6.82 (d, 4H), 7.32 (d, 4H), 7.42 (d, 2H), 7.85 (d, 2H); ESIMS, *m*/*z* for C₂₈H₂₈O₃N₂ [M+H]⁺: 441.

### Example 103

2-[4-(3-ethoxy-trans-1-propen-1-yl)phenyl]-4,5-bis (4-diethoxyphenyl) imidazole: 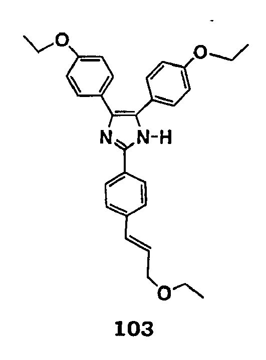 ¹H NMR (400 MHz, CD₃SOCD₃) δ 1.18 (t, 3H), 1.32 (t, 6H), 3.52 (q, 2H), 3.96 (q, 4H), 4.10 (d, 2H), 6.33 (m, 1H), 6.61 (d, 1H), 6.81 (d, 4H), 7.31 (d, 4H), 7.45 (d, 2H), 7.86 (d, 2H); ESIMS, *m*/*z* for C₃₀H₃₂O₃N₂ [M+H]⁺: 469.

### Example 104

2-[4-(3-ethoxy-trans-1-propen-1-yl)phenyl]-4,5-bis (4-diisopropyloxyphenyl) imidazole: 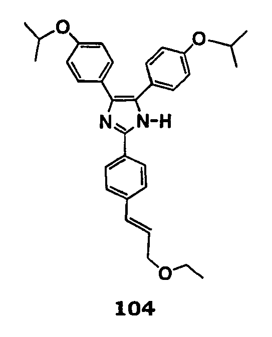 ¹H NMR (400 MHz, CD₃OD) δ 1.18 (t, 3H), 1.25 (d, 12H), 3.51 (q, 2H), 4.10 (d, 2H), 4.53 (m, 2H), 6.32 (m, 1H), 6.60 (d, 1H), 6.80 (d, 4H), 7.31 (d, 4H), 7.43 (d, 2H), 7.86 (d, 2H); ESIMS, *m*/*z* for C₃₂H₃₆O₃N₂ [M+H]⁺: 497.

### Example 105

1-(3-imidazol-1-yl-propyl)-2-[4-(3-ethoxy-trans-1-propen-1-yl)phenyl]-4,5-bis (4-dimethoxyphenyl) imidazole: 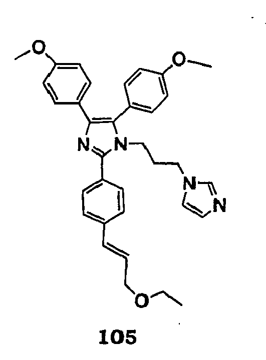 ¹H NMR (400 MHz, CD₃OD) δ 1.20 (t, 3H), 1.75 (m, 2H), 3.55 (q, 2H), 3.68 (m, 5H), 3.82 (s, 3H), 3.88 (t, 2H), 4.14 (d, 2H), 6.41 (m, 1H), 6.70 (m, 5H), 6.97 (d, 2H), 7.21 (d, 2H), 7.28 (d, 2H), 7.30 (s, 1H), 7.50 (s, 4H); ESIMS, *m*/*z* for C₃₄H₃₆O₃N₄ [M+H]⁺: 549.

### Example 106

1-(3-imidazol-1-yl-propyl)-2-[4-(3-ethoxy-trans-1-propen-1-yl)phenyl]-4,5-bis (4-diethoxyphenyl) imidazole: 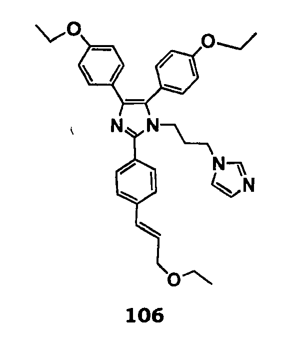 ¹H NMR (400 MHz, CD₃OD) δ 1.20 (t, 3H), 1.29 (t, 3H), 1.39 (t, 3H), 1.74 (m, 2H), 3.55 (q, 2H), 3.66 (t, 2H), 3.86 (t, 2H), 3.91 (q, 2H), 4.04 (q, 2H), 4.14 (d, 2H), 6.41 (m, 1H), 6.67 (m, 5H), 6.94 (d, 2H), 7.18 (d, 2H), 7.27 (d, 2H), 7.31 (s, 1H), 7.50 (s, 4H); ESIMS, *m*/*z* for C₃₆H₄₀O₃N₄ [M+H]⁺: 577.

### Example 107

1-(3-imidazol-1-yl-propyl)-2-[4-(3-ethoxy-trans-1-propen-1-yl)phenyl]-4,5-bis (4-diisopropyloxyphenyl) imidazole: 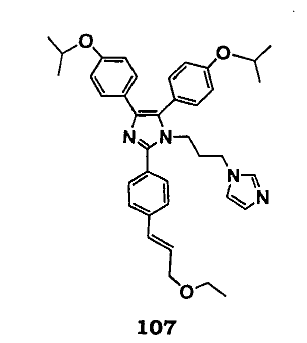 ¹H NMR (400 MHz, CD₃OD) δ 1.20 (t, 3H), 1.20 (d, 6H), 1.30 (d, 6H), 1.74 (m, 2H), 3.54 (q, 2H), 3.65 (t, 2H), 3.85 (t, 2H), 4.14 (d, 2H), 4.47 (m, 1H), 4.60 (m, 1H), 6.40 (m, 1H), 6.67 (m, 5H), 6.93 (d, 2H), 7.17 (d, 2H), 7.28 (d, 2H), 7.34 (s, 1H), 7.50 (s, 4H); ESIMS, *m*/*z* for C₃₈H₄₄O₃N₄ [M+H]⁺: 605.

### Method C-2

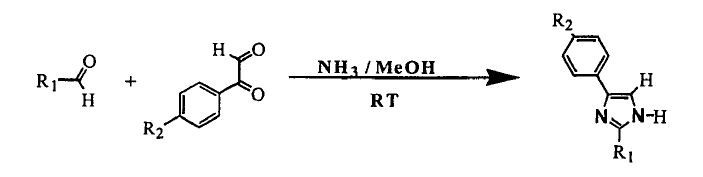

Imidazoles of this type were synthesized in the following way:
The appropriate substituted phenylglyoxal and aldehyde (equal molar amount to the phenylglyoxal)) were stirred in methanolic ammonia at room temperature (TLC mornitored). At completion, the reaction mixture diluted with ethyl acetate and washed with water (X2). The organic layer was then washed with hydrochloric acid (2N) until no more desired compound in the organic layer. The aqueous layer was then neutralized with aqueous NaOH (2 N), and extracted with CH₂CL₂ (X2). The organic layer was dried (Na₂SO₄) and evaporated. Further chromatographic purification then gave the desired compound. The following compounds were synthesized in this way:

### Example 108

2-[4-(3-ethoxy-trans-1-propen-1-yl)phenyl]-4-(4-N,N-diethylphenyl) imidazole: 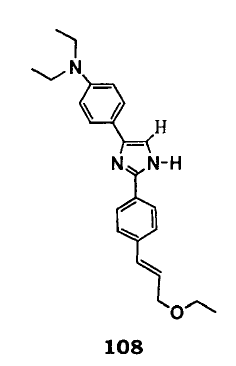 ¹H NMR (400 MHz, CD₃OD) δ 1.10 (t, 6H), 1.17 (t, 3H), 3.30 (q, 4H), 3.49 (q, 2H), 4.08 (d, 2H), 6.31 (m, 1H), 6.58 (d, 1H), 6.68 (d, 2H), 7.18 (s, 1H), 7.42 (d, 2H), 7.50 (d, 2H), 7.82 (d, 2H); ESIMS, *m*/*z* for C₂₄H₂₉ON₃ [M+H]⁺: 376.

### Example 109

2-[4-(3-ethoxy-trans-1-propen-1-yl)phenyl]-4-(4-methoxyphenyl) imidazole: 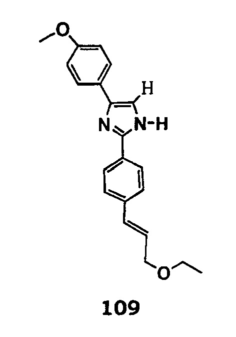 ¹H NMR (400 MHz, CD₃OD) δ 1.18 (t, 3H), 3.51 (q, 2H), 3.78 (s, 3H), 4.08 (d, 2H), 6.32 (m, 1H), 6.59 (d, 1H), 6.90 (d, 2H), 7.29 (s, 1H), 7.44 (d, 2H), 7.62 (d, 2H), 7.82 (d, 2H); ESIMS, *m*/*z* for C₂₁H₂₂O₂N₂ [M+H]⁺: 335.

### Method C-3

### Example 111

2-[4-trans-(2-N,N-dimethylcarbonyl)-ethenyl]phenyl}-4,5-bis (4-N,N-dimethylaminophenyl) imidazole:

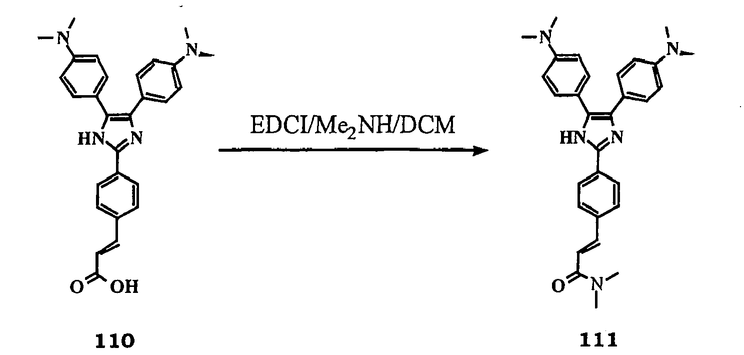

To a solution of compound **110**(100 mg, 0.22 mmol) in dichloromethane (5.0 mL) was added dimethylamine hydrochloride (54 mg, 0.66 mmol), EDCI (51 mg, 0.26 mmol), and DMAP (40 mg, 0.33 mmol). After stirring overnight at room temperature (23°C), the solution was diluted with ethyl acetate and washed with water. The organic layer was dried (Na₂SO₄), and evaporated. The residue was purified via preparative TLC the give the desired compound as a yellow solid. Compound **111** has¹H NMR (400 MHz, CD₃OD) δ 2.92 (s, 12H), 3.03 (s, 3H), 3.22 (s, 3H), 6.72 (d, 4H), 7.16 (d, 1H), 7.32 (d, 4H), 7.55 (d, 1H), 7.75 (d, 2H), 7.96 (d, 2H); ESIMS, *m*/*z* for C₃₀H₃₃ON₅ [M⁺H]⁺: 480.

### Method C-4

### Example 113

2-[4-(3-hydroxy-trans-1-propen-1-yl)phenyl]-4,5-bis (4-N,N-dimethylaminophenyl) imidazole: 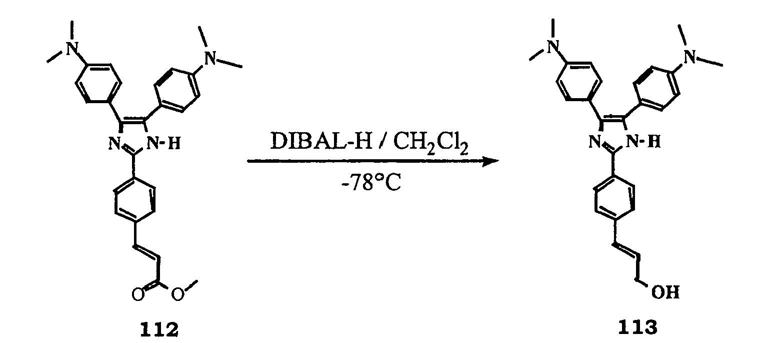 DIBAL-H (1.0 M in DCM, 1.76 ml, 1.76 mmol) was added dropwise to a solution of compound **112** DCM (207 mg, 0.44 mmol) at -78°C. After 1 h at - 78°C, aqueous sodium hydroxide (1.0 M, 20 mL) was added and the mixture was warmed to 23°C. Layers were separated and the aqueous layer was extracted with DCM (X2). The combined organic layers were dried (Na₂SO₄) and evaporated. Purification on preparative TLC gave the desired compound **113** 159 mg, as a yellow solid. Compound **113** has: ¹H NMR (400 MHz, CD₃OD) δ 2.90 (s, 12H), 4.20 (d, 2H), 6.38 (m, 1H), 6.59 (d, 1H), 6.67 (d, 4H), 7.28 (d, 4H), 7.43 (d, 2H), 7.85 (d, 2H); ESIMS, *m*/*z* for C₂₈H₃₀ON₄ [M+H]⁺: 439.

### Method C-5

### Example 114

1-methyl-2-[4-(3-hydroxy-trans-1-propen-1-yl)phenyl]-4,5-bis (4-N,N-dimethylaminophenyl) imidazole: 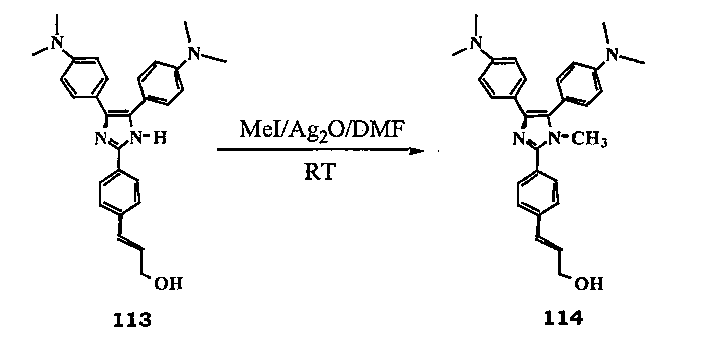 A suspention of compound **113**(11 mg, 0.026 mmol), methyliodide (31 mL, 0.031 mmol), silver oxide (excess) in DMF was stirred at 23°C overnight. The mixture was then diluted with DCM and washed with water.). The organic layer was dried (Na₂SO₄) and evaporated. Purification on preparative TLC gave the desired compound, 4.0 mg, as a yellow solid. Compound **114** has: ¹H NMR (400 MHz, CD₃OD) δ 2.84 (s, 6H), 2.96 (s, 6H), 3.45 (s, 3H), 4.23 (d, 2H), 6.45 (m, 1H), 6.63 (d, 2H), 6.66 (d, 1H), 6.80 (d, 2H), 7.15 (d, 2H), 7.27 (d, 2H), 7.53 (d, 2H), 7.63 (d, 2H); ESIMS, *m*/*z* for C₂₉H₃₂ON₄ [M+H]⁺: 453.

### Method C-6

### Example 115

2-[4-(3-pivalate-trans-1-propen-1-yl)phenyl]-4,5-bis (4-N,N-dimethylaminophenyl) imidazole: 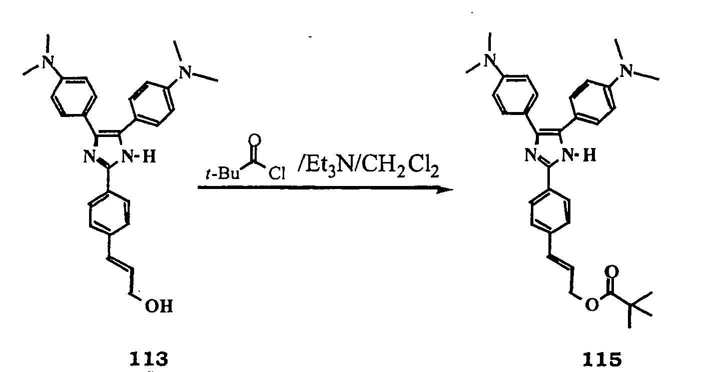 Compound **114** was prepared via acylation of allylic alcohol **113** A mixture of pivaloyl chloride (0.1 M) and triethylamine (0.15 M) (0.55 mL) was added to a solution of allylic alcohol **113** (20 mg, 0.045 mmol) in DCM. at -20°C. The resulting mixture was stirred for 30 min, diluted with DCM, washed with water. The organic layer was dried (Na₂SO₄) and evaporated. Purification on preparative TLC gave the desired compound **115** 5 mg, as a yellow solid. Compound **115** has: ¹H NMR (400 MHz, CD₃OD) δ 1.10 (s, 9H), 2.90 (s, 12H), 4.70 (d, 2H), 6.34 (m, 1H), 6.65 (d, 1H), 6.67 (d, 4H), 7.30 (d, 4H), 7.48 (d, 2H), 7.85 (d, 2H); ESIMS, *m*/*z* for C₃₃H₃₈O₂N4 [M+H]⁺: 523.

### Method C-7

### Examples 116

2-[4-(3-methylcarbonyl-trans-1-propen-1-yl)phenyl]-4,5-bis (4-N,N-dimethylaminophenyl) imidazole: 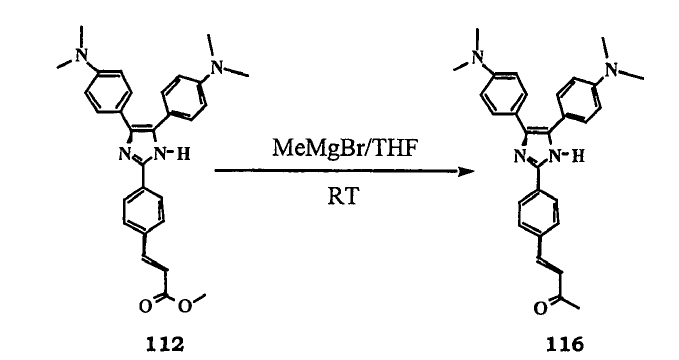 Methylmagnesium bromide (1.0 M, in dibutylether, 0.63 mL, 0.65 mmol) was added to solution of compound **112** in THF (5.0 mL) at -78 °C. After stirring overnight under argon, during which time the mixture was warmed to 23°C, it was diluted with water and extracted with DCM.. The combined organic layers were dried (Na₂SO₄) and evaporated. Purification on preparative TLC gave the desired compound as a yellow solid. Compound 116 has: ¹H NMR (400 MHz, CD₃OD) δ 2.36 (s, 3H), 2.92 (s, 12H), 6.71 (d, 4H), 6.80 (d, 1H), 7.31 (d, 4H), 7.64 (d, 1H), 7.70 (d, 2H), 7.98 (d, 2H); ESIMS, *m*/*z* for C₂₉H₃₀ON₄ [M⁺H]⁺: 451.

### Method C-8

### Example 118

2-[4-(3-methylcarbonyl-trans-1-propen-1-yl)phenyl]-5-methoxy benzimidazole: 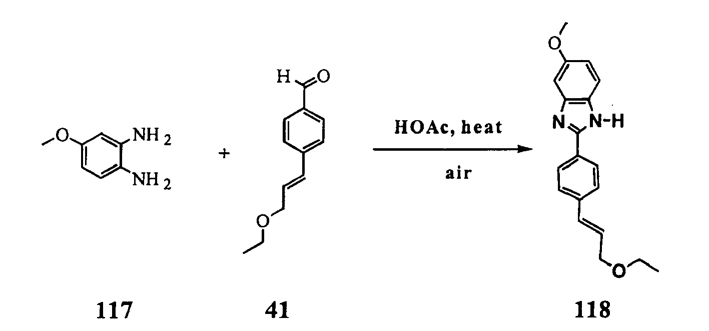 Compound **118** was prepared according to a known procedure (Lee, M. et al Med. Chem.Res. 1993, 2, 79-86). Compound **118** has: ¹H NMR (400 MHz, CD₃OD) δ 1.20 (t, 3H), 3.54 (q, 2H), 3.81 (s, 3H), 4.13 (d, 2H), 6.41 (m, 1H), 6.66 (d, 1H), 6.86 (m, 1H), 7.04 (d, 1H), 7.44 (d, 1H), 7.53 (d, 2H), 7.96 (d, 2H); ESIMS, *m*/*z* for C₁₉H₂₀O₂N₂ [M+H]⁺: 309.

### Example 119

2-[4-(3-ethoxy-trans-1-propen-1-yl)phenyl]-4,5-bis-(4-N-isopropylaminophenyl) imidazole,

### Compound 119 was prepared according to method C-1 by using the appropriate starting materials.

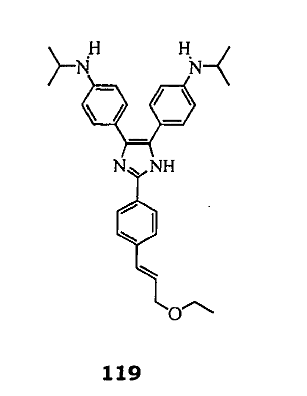

¹H NMR (400 MHz, CD₃OD) δ 1.15 (d, 6H), 1.16 (d, 6H), 1.18 (t, 3H), 3.53 (m, 4H), 4.10 (br s, 2H), 6.33 (br s, 1H), 6.56 (m, 5H), 7.23 (d, 4H), 7.44 (d, 2H), 7.85 (d, 2H); ESIMS, *m*/*z* for C₃₂H₃₈ON₄ [M+H]⁺: 495.

### Example 120

2-[4-(3-ethoxy-trans-1-propen-1-yl)phenyl]-4-(4-N-ethylaminophenyl)-5-(4-N-isopropylaminophenyl) imidazole,

### Compound 120 was prepared according to method C-1 by using the

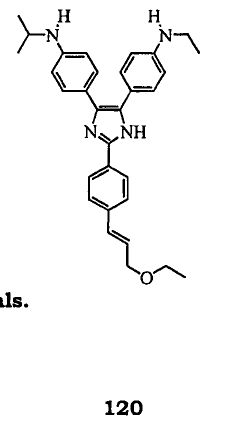

**appropirate starting materials.**
¹H NMR (400 MHz, CD₃OD) δ 1.16 (m, 12H), 3.07 (q, 2H), 3.53 (m, 3H), 4.10 (d, 2H), 6.33 (m, 1H), 6.56 (m, 5H), 7.23 (d, 4H), 7.44 (d, 2H), 7.85 (d, 2H); ESIMS, *m*/*z* for C₃₁H₃₆ON₄ [M+H]⁺: 481.

### Example 121

2-[4-(3-ethoxy-trans-1-propen-1-yl)phenyl]-4-(4-fluorophenyl)-5-(4-N-isopropylaminophenyl) imidazole,

### Compound 121 was prepared according to method C-1 by using the appropriate starting materials.

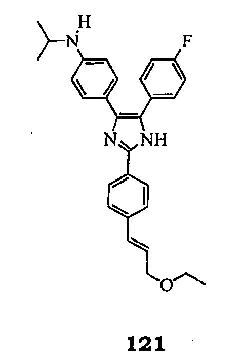

¹H NMR (400 MHz, CD₃OD) δ 1.16 (m, 9H)), 3.52 (m, 3H), 4.09 (d, 2H), 6.32 (m, 1H), 6.56 (d, 2H), 6.58 (d, 1H), 6.98 (dd, 2H), 7.14 (d, 2H), 7.45 (m, 4H), 7.85 (d, 2H); ESIMS, *m*/*z* for C₂₉H₃₀FON₃ [M+H]⁺: 456.

### Example 122

2-[4-(3-ethoxy-trans-1-propen-1-yl)phenyl]-4-(4-N,N-dipropylphenyl) imidazole,

### Compound 122 was prepared according to method C-2 by using the appropriate starting materials.

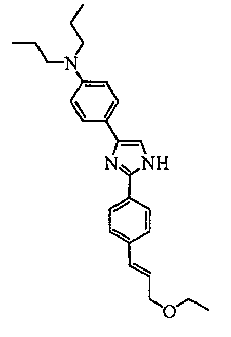

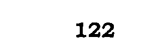

¹H NMR (400 MHz, CD₃OD) δ 0.91 (t, 6H), 1.19 (t, 3H), 1.58 (m, 4H), 3.26 (m, 4H), 3.53 (m, 2H), 4.11 (d, 2H), 6.35 (m, 1H), 6.63 (d, 1H), 6.66 (d, 1H), 7.19 (s, 1H), 7.48 (m, 4H), 7.83 (d, 2H); ESIMS, *m*/*z* for C₂₆H₃₃ON₃ [M+H]⁺: 404.

### Example 123

2-[4-(3-ethoxy-trans-1-propen-1-yl)phenyl]-4-(4-N-isopropylphenyl) imidazole,

### Compound 123 was prepared according to method C-2 by using the appropriate starting materials.

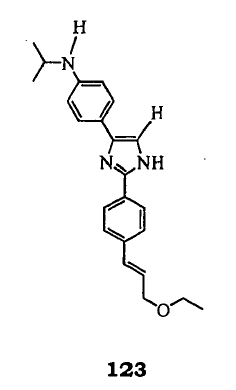

¹H NMR (400 MHz, CD₃OD) δ 1.16 (d, 6H), 1.19 (t, 3H), 3.53 (q, 2H), 3.60 (m, 1H), 4.11 (d, 2H), 6.35 (m, 1H), 6.62 (d, 1H), 6.64 (d, 2H), 7.19 (s, 1H), 7.46 (m, 4H), 7.83 (d, 2H); ESIMS, *m*/*z* for C₂₃H₂₇ON₃ [M+H]⁺: 362.

### Example 124

2-[4-(3-ethoxy-trans-1-propen-1-yl)phenyl]-4-(4-N-isobutylphenyl) imidazole,

### Compound 124 was prepared according to method C-2 by using the appropriate starting materials.

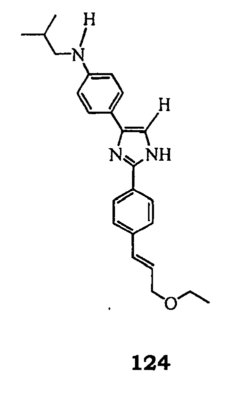

¹H NMR (400 MHz, CD₃OD) δ 0.94 (d, 6H), 1.19 (t, 3H), 1.87 (m, 1H), 2.90 (d, 2H), 3.53 (q, 2H), 4.12 (d, 2H), 6.35 (m, 1H), 6.62 (m, 3H), 7.18 (s, 1H), 7.46 (m, 4H), 7.83 (d, 2H); ESIMS, *m*/*z* for C₂₄H₂₉ON₃ [M+H]⁺: 376.

### Example 125

2-[4-(3-ethoxy-trans-1-propen-1-yl)phenyl]-4-(4-N-morpholinophenyl) imidazole,

### Compound 125 was prepared according to method C-2 by using the appropriate starting materials.

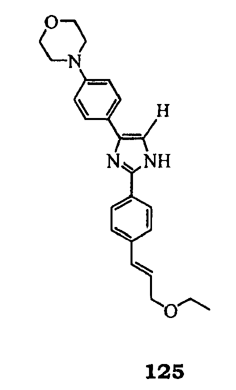

¹H NMR (400 MHz, CD₃OD) δ 1.19 (t, 3H), 3.13 (t, 4H), 3.53 (q, 2H), 3.80 (t, 4H), 4.12 (d, 2H), 6.35 (m, 1H), 6.63 (d, 1H), 6.97 (d, 2H), 7.29 (s, 1H), 7.47 (d, 2H), 7.61 (d, 2H), 7.84 (d, 2H); ESIMS, *m*/*z* for C₂₄H₂₇O₂N₃ [M+H]⁺: 390.

### Example 126

2-[4-(3-ethoxy-trans-1-propen-1-yl)phenyl]-4-[4-N-(N'-ethyl)-piperizanophenyl) imidazole,

### Compound 126 was prepared according to method C-2 by using the appropriate starting material

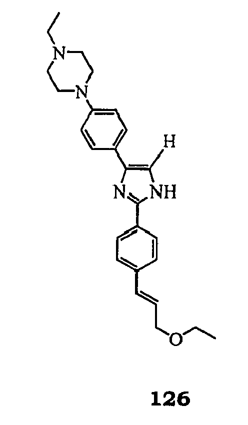

¹H NMR (400 MHz, CD₃OD) δ 1.16 (m, 6H), 2.61 (q, 2H), 2.78 (t, 4H), 3.26 (t, 4H), 3.54 (q, 2H), 4.12 (d, 2H), 6.36 (m, 1H), 6.64 (d, 1H), 7.00 (d, 2H), 7.30 (s, 1H), 7.48 (d, 2H), 7.62 (d, 2H), 7.84 (d, 2H); ESIMS, *m*/*z* for C₂₆H₃₂ON₄ [M+H]⁺: 417.

### Example 127

2-[4-(3-ethoxy-trans-1-propen-1-yl)phenyl]-4-(4-N-morpholinophenyl)-5-methyl-imidazole.

### Compound 127 was prepared according to method C-1 by using the appropriate starting material

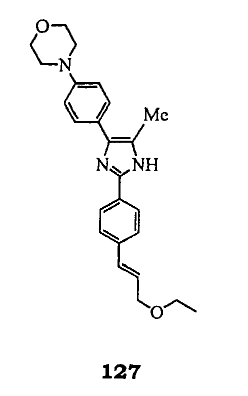

¹H NMR (400 MHz, CD₃OD) δ 1.19 (t, 3H), 2.34 (s, 3H), 3.11 (t, 4H), 3.52 (q, 2H), 3.79 (t, 4H), 4.10 (d, 2H), 6.34 (m, 1H), 6.60 (d, 1H), 6.98 (d, 2H), 7.44 (m, 4H), 7.80 (d, 2H); ESIMS, *m*/*z* for C₂₅H₂₉O₂N₃ [M+H]⁺: 404.

The compounds described herein are capable of sensitizing multi-drug resistant tumor cells to antitumor chemotherapeutic agents, such as doxorubicin and vinblastine. They also have the ability to potentiate the sensitivity of tumor cells susceptible to these chemotherapeutic agents. This invention also relates to a medicament for sensitizing multidrug-resistant tumor cells to antitumor chemotherapeutic agents. It also relates to a medicament for increasing the sensitivity of drug-susceptible tumor cells to antitumor chemotherapeutic agents. In addition, this invention relates to a medicament for preventing the emergence of MDR tumor cells during a course of treatment with antitumor chemotherapeutic agents. Finally, this invention relates to a medicament for reducing the effective dosage of an antitumor chemotherapeutic agent during a course of treatment. It has been found that compounds of Formula 1 have the ability to increase the sensitivity of MDR mammalian cells in culture.

Cytotoxic drugs are commonly used as antitumor chemotherapeutic agents. These agents are also called antiproliferative agents. The desired effect of cytotoxic drugs is selective cell death with destruction of the malignant neoplastic cells and relative sparing of normal cells.

Cytotoxic drugs have also proved valuable in the treatment of other neoplastic disorders including connective or autoimmune diseases, metabolic disorders, dermatological diseases, and DNA virus infections.

Proper use of cytotoxic drugs requires a thorough familiarity with the natural history and pathophysiology of the disease before selecting the cytotoxic agent, determining a dose, and undertaking therapy. Each patient must be carefully evaluated, with attention directed toward factors which may potentiate toxicity, such as overt or occult infections, bleeding dyscrasias, poor nutritional status, and severe metabolic disturbances. In addition, the functional condition of certain major organs, such as liver, kidneys, and bone marrow, is extremely important. Therefore, the selection of the appropriate cytotoxic agent and devising an effective therapeutic regimen is influenced by the presentation of the patient.

Cytotoxic drugs as antitumor chemotherapeutic agents can be subdivided into several broad categories, including, (1) alkylating agents, such as mechlorethamine, cyclophosphamide, melphalan, uracil mustard, chlorambucil, busulfan, carmustine, lomustine, semustine, streptozoticin, and decrabazine; (2) antimetabolites, such as methotrexate, fluorouracil, fluorodeoxyuridine, cytarabine, azarabine, idoxuridine, mercaptopurine, azathioprine, thioguanine, and adenine arabinoside; (3) natural product derivatives, such as vinblastine, vincristine, dactinomycin, daunorubicin, doxorubicin, mithramycin, bleomycin, etoposide, teniposide, and mitomycin-C; and (4) miscellaneous agents, such as hydroxyurea, procarbezine, mititane, and cis-platinum.

Important antitumor chemotherapeutic agents (with the usual effective dosage) to which clinical multidrug-resistance has been observed include vinblastine (0.1 mg per kilogram per week), vincristine (0.01 mg per kilogram per week), etoposide (35 to 50 mg per square meter per day), dactinomycin (0.15 mg per kilogram per day), doxorubicin (500 to 600 mg per square meter per week), daunorubicin (65 to 75 mg per square meter per week), and mithramycin (0.025 mg per kilogram per day). MDR has been shown to occur *in vitro* as well as in the clinic.

Multidrug-resistant cell lines are easily obtainable for *in vitro* determination of drug sensitization by compounds of the present invention. *In vitro* potentiation of antineoplastic cytotoxicity by the imidazole derivatives of the present invention was measured in both CEM/VLB1000 and SK/VLB1000 cell lines. The multidrug resistant cell lines were obtained from Dr. Victor Ling, Ontario Cancer Institute, Toronto, Canada. The CEM/VLB 1000 cell line was maintained as a suspension in minimum essential medium supplemented with 10% fetal bovine serum in a humidied atmosphere of 95% air and 5% CO₂ while the SK/VLB 1000 cell line was maintained as adherent cells using the identical medium conditions as the CEM cells. The CEM/VLB 1000 cells were seeded at a density of 5 x 10⁴ cells/well in a 96 well microtiter plate while the SK/VLB 1000 cell line was seeded at a density of 2,500 cells/well after trypsinization. Vinblastine (5 µg/mL, for the CEM cells) or Taxol (3 µg/mL, for the SK cells) and compound (0.01 to 50 µM) were added directly to the wells. After an incubation of 48 hours in presence of drug, alamar blue (B. Page et al., Int. J. Oncol. 3: 473-476, 1993) was added (10 µL to the 200 µL cell suspension) for a period of 24 hours after which the fluorescence (excitation = 530 nM, emission = 590 nM) was read for each well using a "CytoFluor" microtiter fluorometer plate reader. This assay measures the effective concentration of compound necessary to enhance the cytotoxicity (EC₅₀) of vinblastine in the MDR cell line. The compounds of the present invention had EC₅₀ values in the range of 0.06 to 10 µM.

³H-vinblastine accumulation was also measured in the CEM/VLB1000 cell line. Coming Easy-Wash 96 well plates were pretreated with PBS and 1% BSA for 60 minutes and then removed. CEM/VLB1000 cells were seeded at 2 x 10⁵, 40 µL volume. Plates were incubated at 37°C for 30-60 minutes prior to use. The reference reversing agent, verapamil, or the compound of the present invention was added to the well followed by addition of media containing ³H-vinblastine (final concentration = 275 nM). Plates were allowed to incubate for 3 hours at 37°C. Cells were harvested onto pretreated Wallace filtermats A (pretreated with 0.1% polyethyleneimine) using a TomTek harvester-96. After filtering, the filtermats were allowed to dry completely. Meltix B scintillant was then added to the filtermats. The filters were then placed in a 90°C oven for approximately 3-5 minutes and then removed. Scintillant was allowed to solidify on the filtermats. Filtermats were then placed in sample bags and read on a Wallace BetaPlate scintillation counter. The effects of compounds of the present invention in the cytotoxicity potentiation assays and vinblastine (VLB) accumulation assay are given in the Table below:

| Examples | Cytotoxicity Potentiation (µM)¹ CEM/VLB1000 | [³H]VLB Accumulation (µM)² CEM/VLB1000 |
|---|---|---|
| **56** | 0.55 | NT³ |
| **57** | 0.21 | NT |
| **58** | 0.47 | NT |
| **59** | 0.55 | NT |
| **60** | 0.45 | NT |
| **61** | 0.16 | NT |
| **62** | 1.03 | NT |
| **63** | 0.32 | NT |
| **64** | 0.55 | NT |
| **65** | 0.25 | NT |
| **66** | 0.85 | NT |
| **67** | 0.098 | NT |
| **68** | 0.39 | NT |
| **69** | 0.33 | NT |
| **70** | 0.50 | NT |
| **71** | 0.37 | NT |
| **72** | 0.32 | NT |
| **73** | 0.34 | NT |
| **74** | 0.13 | 2.0 |
| **75** | 0.098 | 1.2 |
| **76** | 0.11 | NT |
| **77** | 0.34 | 1.2 |
| **78** | 0.45 | NT |
| **79** | 1.21 | NT |
| **80** | 0.88 | NT |
| **81** | 0.32 | NT |
| **82** | 0.96 | NT |
| **83** | 1.30 | NT |
| **84** | 0.09 | NT |
| **85** | 0.11 | NT |
| **86** | 0.26 | NT |
| **87** | 0.06 | NT |
| **88** | 0.26 | NT |
| **89** | 0.24 | NT |
| **90** | 0.12 | NT |
| **91** | 0.11 | NT |
| **92** | 0.22 | NT |
| **93** | 0.15 | NT |
| **94** | 0.25 | NT |
| **95** | 0.05 | NT |
| **96** | 0.73 | NT |
| **97** | 0.19 | NT |
| **98** | 0.60 | NT |
| **99** | 0.29 | NT |
| **100** | 3.4 | NT |
| **101** | 0.2 | NT |
| **102** | 0.35 | NT |
| **103** | 0.24 | NT |
| **104** | 0.25 | NT |
| **105** | 0.65 | NT |
| **106** | 0.38 | NT |
| **107** | 0.49 | NT |
| **108** | 0.30 | NT |
| **109** | 1.85 | NT |
| **111** | 0.62 | 1.7 |
| **113** | 0.41 | 3.9 |
| **114** | 0.63 | NT |
| **115** | 0.48 | NT |
| **116** | 0.23 | NT |
| **118** | 1.00 | NT |
| **119** | .067 | NT |
| **120** | .053 | NT |
| **121** | .12 | NT |
| **122** | .28 | NT |
| **123** | .34 | NT |
| **124** | .25 | NT |
| **125** | .37 | NT |
| **126** | .71 | NT |
| **127** | .33 | NT |

| | | |
|---|---|---|
| ¹Values presented are the midpoint (EC₅₀) of the minimum and maximum cytotoxicity induced by 3-5 µg/mL vinblastine and the specific compound of the present invention. ²Values presented are the midpoint (EC₅₀) of the minimum and maximum increase in accumulation of ³H-vinblastine caused by the specific compound of the present invention. ³NT = Not tested. | | |

The modulation of multidrug-resistance demonstrated by the imidazole derivatives described herein provides a medicament for the treatment of multidrug-resistant tumors. The multidrug-resistance modulatory properties of the compounds described herein also provides a medicament for the prevention of the emergence of multi-drug resistant tumors during the course of cancer treatment. These same compounds additionally provide a medicament for reducing the required dosage of an antitumor chemotherapeutic agent.

The use of the compounds of formula I involve (1) the administration of a compound of Formula 1 prior to, together with, or subsequent to the administration of an antitumor chemotherapeutic agent; and (2) the administration of a combination of a compound of Formula 1 and an antitumor chemotherapeutic agent.

Thus, the compounds of Formula 1 are useful in the treatment of multidrug-resistant tumor cells or tumor cells in general, either separately or in combination with an antitumor chemotherapeutic agent. These compounds may be administered orally, topically or parenterally in dosage unit formulations containing conventional non-toxic pharmaceutically acceptable carriers, adjuvants, and vehicles. The term parenteral as used herein includes subcutaneous injections, intravenous, intramuscular, intrasternal injection or infusion techniques.

The present invention also has the objective of providing suitable topical, oral, and parenteral pharmaceutical formulations for use as medicaments of the present invention. The compounds of the present invention may be administered orally as tablets, aqueous or oily suspensions, lozenges, troches, powders, granules, emulsions, capsules, syrups or elixirs. The composition for oral use may contain one or more agents selected from the group of sweetening agents, flavouring agents, colouring agents and preserving agents in order to produce pharmaceutically elegant and palatable preparations. The tablets contain the acting ingredient in admixture with non-toxic pharmaceutically acceptable excipients which are suitable for the manufacture of tablets. These excipients may be, for example, (1) inert diluents , such as calcium carbonate, lactose, calcium phosphate or sodium phosphate; (2) granulating and disintegrating agents, such as corn starch or alginic acid; (3) binding agents, such as starch, gelatin or acacia; and (4) lubricating agents, such as magnesium stearate, stearic acid or talc. These tablets may be uncoated or coated by known techniques to delay disintegration and absorption in the gastrointestinal tract and thereby provide a sustained action over a longer period. For example, a time delay material such as glyceryl monostearate or glyceryl distearate may be employed. Coating may also be performed using techniques described in the U.S. Patent Nos. 4,256,108; 4,160,452; and 4,265,874 to form osmotic therapeutic tablets for control release.

Formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, such as peanut oil, liquid paraffin or olive oil.

Aqueous suspensions normally contain the active materials in admixture with excipients suitable for the manufacture of aqueous suspension. Such expicients may be (1) suspending agent such as sodium carboxymethyl cellulose, methyl cellulose, hydroxypropylmethylcellulose, sodium alginate, polyvinylpyrrolidone, gum tragacanth and gum acacia; (2) dispersing or wetting agents which may be (a) naturally occurring phosphatide such as lecithin; (b) a condensation product of an alkylene oxide with a fatty acid, for example, polyoxyethylene stearate; (c) a condensation product of ethylene oxide with a long chain aliphatic alcohol, for example, heptadecaethylenoxycetanol; (d) a condensation product of ethylene oxide with a partial ester derived from a fatty acid and hexitol such as polyoxyethylene sorbitol monooleate, or (e) a condensation product of ethylene oxide with a partial ester derived from fatty acids and hexitol anhydrides, for example polyoxyethylene sorbitan monooleate.

The pharmaceutical compositions may be in the form of a sterile injectable aqueous or oleagenous suspension. This suspension may be formulated according to known methods using those suitable dispersing or wetting agents and suspending agents which have been mentioned above. The sterile injectable preparation may also a sterile injectable solution or suspension in a non-toxic parenterally-acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose, any bland fixed oil may be employed including synthetic mono- or diglycerides. In adition, fatty acids such as oleic acid find use in the preparation of injectables.

A compound of Formula 1 may also be administered in the form of suppositories for rectal administration of the drug. These compositions can be prepared by mixing the drug with a suitable non-irritating excipient which is solid at ordinary temperature but liquid at the rectal temperature and will therefore melt in the rectum to release the drug. Such materials are cocoa butter and polyethylene glycols.

The compounds of the present invention may also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

For topical use, creams, ointments, jellies, solutions or suspensions, etc., containing the compounds of Formula 1 are employed.

Dosage levels of the compounds of the present invention are of the order of about 0.5 mg to about 100 mg per kilogram body weight, with a preferred dosage range between about 20 mg to about 50 mg per kilogram body weight per day (from about 25 mg to about 5 gms per patient per day). The amount of active ingredient that may be combined with the carrier materials to produce a single dosage will vary depending upon the host treated and the particular mode of administration. For example, a formulation intended for oral administration to humans may contain 5 mg to 1 g of an active compound with an appropriate and convenient amount of carrier material which may vary from about 5 to about 95 percent of the total composition. Dosage unit forms will generally contain between from about 5 mg to about 500 mg of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

In addition, some of the compounds of the instant invention may form solvates with water or common organic solvents. Such solvates are encompassed within the scope of the invention.

## Claims

1. **Use of a therapeutically effective amount of a** compound of Formula 1, 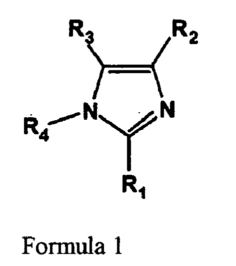 **and a pharmaceutically acceptable carrier for the manufacture of a medicament for treatment of tumor cells, wherein the medicament is administered prior to, together with, or subsequent to the administration of an antitumor chemotherapeutic agent, wherein for the compound of Formula 1, wherein** the substituents R₁, R₂, R₃, and R₄ are defined as described in **A** and **B** below:
A. when R₁ is selected from the group consisting of:
(i) substituted C₁₋₁₁ alkyl or substituted C₂₋₁₁ alkenyl, wherein the substituents are selected from the group consisting of hydroxy, C₁₋₆ alkyloxy; or
(ii) mono-, di-, and tri-substituted aryl-C₀₋₁₁ alkyl wherein aryl is selected from the group consisting of phenyl, furyl, thienyl wherein the substituents are selected from the group consisting of:
(a) phenyl, *trans* -2-phenylethenyl, 2-phenylethynyl, 2-phenylethyl, wherein the phenyl group is mono- or disubstituted with a member selected from the group consisting of hydroxy, halo, C₁₋₄ alkyl and C₁₋₄ alkyloxy,
(b) substituted C₁₋₆ alkyl, substituted C₂₋₆ alkyloxy, substituted C₂₋₆ alkylthio, or substituted C₂₋₆ alkoxycarbonyl, wherein the substituents are selected from the group consisting of C₁₋₆ alkoxy, and C₁₋₆ alkylthio; and
(c) C₁₋₁₁ CO₂R₅, C₁₋₁₁ CONHR₅, *trans*- CH=CHCO₂R₅, or *trans-*CH=CHCONHR₅ wherein R₅ is C₁₋₁₁ alkyl, or phenyl C₁₋₁₁ alkyl, C₁₋₆ alkoxycarbonylmethyleneoxy;
then R₂ and R₃ are each independently selected from the group consisting of mono-, di-, and tri-substituted phenyl wherein the substituents are independently selected from:
(i) substituted C₁₋₆ alkyl,
(ii) substituted C₁₋₆ alkyloxy, C₃₋₆ alkenyloxy, substituted C₃₋₆ alkenyloxy,
(iii) substituted C₁₋₆ alkyl-amino, di(substituted C₁₋₆ alkyl)amino,
(iv) C₃₋₆ alkenyl-amino, di(C₃₋₆ alkenyl)amino, substituted C₃₋₆ alkenyl-amino, or di(substituted C₃₋₆ alkenyl)amino,
(v) pyrrolidino, piperidino, morpholino, imidazolyl, substituted imidazolyl, piperazino, 4-N-C₁₋₆ alkylpiperazino, 4- N-C₃₋₆ alkenylpiperazino, 4-N- (C₁₋₆ alkoxyC₁₋₆ alkyl)piperazino, 4-N-(C₁₋₆ alkoxyC₃₋₆ alkenyl)piperazino, 4-N-(C₁₋₆ alkylaminoC₁₋₆ alkyl)piperazino, 4-N-(C₁₋₆ alkylamino C₃₋₆ alkenyl)piperazino,
wherein the substituents are selected from the group consisting of
(a) hydroxy, C₁₋₆ alkylalkoxy, C₁₋₆ alkylamino;
(b) C₃₋₆ alkenyloxy, C₃₋₆ alkenylamino, or
(c) pyrrolidino, piperidino, morpholino, imidazolyl, substituted imidazolyl, piperazino, 4-N-C₁₋₆ alkylpiperazino, 4-N-C₃₋₆ alkenylpiperazino, 4-N-(C₁₋₆ alkoxy C₁₋₆ alkyl)piperazino, 4-N-(C₁₋₆ alkoxy C₃₋₆ alkenyl)piperazino, 4-N-(C₁₋₆ alkylamino C₁₋₆ alkyl)piperazino, 4-N-(C₁₋₆ alkylamino C₃₋₆ alkenyl)piperazino,
or R₂ and R₃ taken together forming an aryl group **or substituted aryl**, wherein the substituents are defined as above in (i)-(v);
and R₄ is selected from the group consisting of:
(i) hydrogen;
(ii) substituted C₁₋₁₁ alkyl or C₂₋₁₁ alkenyl wherein the substituents are independently selected from the group consisting of hydrogen, hydroxy, C₁₋₆ alkyloxy, C₁₋₆alkylthio, C₁₋₆ alkylamino, phenyl-C₁₋₆ alkylamino, C₁₋₆ alkoxycarbonyl; or
(iii) substituted aryl C₀₋₁₁ alkyl wherein the aryl group is selected from phenyl, imidazolyl, furyl, thienyl in which the substituents are selected from the **group consisting of**:A.(a-c); or
B. when R₁ is selected from the group consisting of:
mono-,di-, and tri-substituted aryl-C₀₋₆ alkyl wherein aryl is selected from the group consisting of phenyl, thienyl, and the substituents are selected from the group consisting of:
(a) *trans*-2-substituted benzimidazolylethenyl, *trans*-2-substituted benzoxazolylethenyl, or *trans*-2-substituted benzthiazolylethenyl, in which the substituents are selected from the group consisting of hydrogen, hydroxy, halo, trihalomethyl, C₁₋₄ alkyl, C₁₋₄ alkyloxy, C₁₋₄ alkyloxycarbonyl, C₁₋₄ alkylamino, di(C₁₋₄ alkyl)amino, C₃₋₆ alkenylamino, di(C₃₋₆ alkenyl)amino, C₁₋₄ akyloxy-C₁₋₄ alkylamino, substituted C₁₋₄ alkyl and C₁₋₄ alkyloxy, substituted C₁₋₄ alkyloxycarbonyl, substituted C₁₋₄ alkylamino, di(substituted C₁₋₄ alkyl)amino, substituted C₃₋₆ alkenylamino, di(substituted C₃₋₆ alkenyl)amino, wherein the substituents are as defined above,
(b) *trans*-2-cyano ethenyl, *trans*-2-alkylsulfonyl ethenyl, *trans-*2 *-* alkenylsulfonyl ethenyl, *trans-2-* substituted alkylsulfonyl ethenyl, *trans*-2- substituted alkenylsulfonyl ethenyl, in which the substituents are as defined above,
(c) C₁₋₆ CO₂R₅, *trans*- CH=CHCO₂R₅, C₁₋₆CONHR₅, or *trans-* CH=CHCONHR₅, wherein R₅ is C₁₋₆ alkoxy C₂₋₆ alkyl, amino C₂₋₆ alkyl, C₁₋₆ alkylamino C₂₋₆ alkyl, di(C₁₋₆ alkyl)amino C₂₋₆ alkyl, C₁₋₆ alkylthio C₂₋₆ alkyl, substituted C₁₋₆ alkoxy C₂₋₆ alkyl, substituted C₁₋₆ alkylamino C₂₋₆ alkyl, di(substituted C₁₋₆ alkyl)amino C₂₋₆ alkyl, substituted C₁₋₆ alkylthio C₂₋₆ alkyl, in which the substituents are selected from the group consisting of pyrrolidino, piperidino morpholino, piperazino, 4-N-C₁₋₆ alkylpiperazino, 4-N-C₃₋₆ alkenylpiperazino, 4-N-(C₁₋₆ alkoxy C₁₋₆ alkyl)piperazino, 4-N-(C₁₋₆ alkoxy C₃₋₆ alkenyl)piperazino, 4-N-(C₁₋₆ alkylamino C₁₋₆ alkyl)piperazino, 4-N-(C₁₋₆ alkylamino C₃₋₆ alkenyl)piperazino, imidazolyl, oxazolyl, thiazolyl,
(d) C₁₋₆CONR₆R₇, or *trans*- CH=CHCONR₆R₇, wherein R₆ and R₇ are independently selected from the group consisting of C₁₋₆ alkyl, phenyl C₁₋₆ alkyl, C₁₋₆ alkoxycarbonylmethyleneoxy, hydroxy C₂₋₆ alkyl, C₁₋₆ alkyloxy C₂₋₆ alkyl, amino C₂₋₆ alkyl, C₁₋₆ alkylamino C₂₋₆ alkyl, di(C₁₋₆ alkyl)amino C₂₋₆ alkyl, C₁₋₆ alkylthio C₂₋₆ alkyl, substituted C₁₋₆ alkoxy C₂₋₆ alkyl, substituted C₁₋₆ alkylamino C₂₋₆ alkyl, di(substituted C₁₋₆ alkyl)amino C₂₋₆ alkyl, substituted C₁₋₆ alkylthio C₂₋₆ alkyl, wherein the substituents are selected from the group consisting of pyrrolidino, piperidino, morpholino, piperazino, 4-N- C₁₋₆ alkylpiperazino, 4-N-C₃₋₆ alkenylpiperazino, 4-N-(C₁₋₆ alkoxy C₁₋₆ alkyl)piperazino, 4-N-(C₁₋₆ alkoxy C₃₋₆ alkenyl)piperazino, 4-N-(C₁₋₆ alkylamino C₁₋₆ alkyl)piperazino, 4-N-(C₁₋₆ alkylamino C₃₋₆ alkenyl)piperazino, imidazolyl, oxazolyl, thiazolyl,
(e) R₇ C(O) C₁₋₆ alkyl, R₇ C(O) C₂₋₆ alkenyl, in which R₇ is defined as above [2(d)],
(f) HO-C₁₋₆ alkyl-C₂₋₆ alkenyl, R₇-O-C₁₋₆ alkyl-C₂₋₆ alkenyl, R₇NH-C₁₋₆ alkyl-C_{2-ó} alkenyl, R₆R₇N-C₁₋₆ alkyl-C₂₋₆ alkenyl, R₇NH-C(O)-O-C₁₋₆ alkyl-C₂₋₆ alkenyl, R₆R₇N-C(O)-O-C₁₋₆ akyl-C₂₋₆ alkenyl, R₇O-C(O)-O-C₁₋₆ alkyl-C₂₋₆ alkenyl, R₇-C(O)-O-C₁₋₆ alkyl-C₂₋₆ alkenyl, wherein R₆ and R₇ **are** defined as above [2(d)],
(g) R₇-O-C₀₋₃ alkyl-C₃₋₆ cycloalkan-1-yl, R₇NH- C₀₋₃ alkyl- C₃₋₆ cycloalkan-1-yl, R₆R₇N- C₀₋₃ alkyl- C₃₋₆ cycloalkan-1-yl, R₇NH-C(O)-O- C₀₋₃ C₃₋₆ cycloalkan-1-yl, R₆R₇N-C(O)-O- C₀₋₃ alkyl- C₃₋₆ cycloalkan-1-yl, R₇O- C(O)-O- C₀₋₃ alkyl- C_{3-ó} cycloalkan-1-yl, R₇-C(O)-O- C₀₋₃ alkyl- C_{3-ó} cycloalkan- 1-yl, R₇O-C(O)-C₀₋₃ alkyl- C₃₋₆ cycloalkan-1-yl, wherein R₇ and **R₆ are** is-defined as above [2(d)];
then R₂ and R₃ are each independently selected from the group consisting of:
(1) hydrogen, halo, trihalomethyl, C₁₋₆ alkyl, substituted C₁₋₆ alkyl, C₂₋₆ alkenyl, substituted C₂₄₋₆ alkenyl, C₁₋₆ alkyloxy, substituted C₁₋₆ alkyloxy, C₃₋₆ alkenyloxy, substituted C₃₋₆ alkenyloxy, C₁₋₆ alkylamino, substituted C₁₋₆ alkylamino, C₃₋₆ alkenylamino, substituted C₃₋₆ alkenylamino,
(2) mono-, di-, and tri-substituted phenyl wherein the substituents are independently selected from:
(i) halo, trifluoromethyl, substituted C₁₋₆ alkyl,
(ii) C₁₋₆ alkyloxy, substituted C₁₋₆ alkyloxy, C₃₋₆ alkenyloxy, substituted C₃₋₆ alkenyloxy,
(iii) C₁₋₆ alkyl-amino, di(C₁₋₆ alkyl)amino, substituted C₁₋₆ alkyl-amino, di(substituted C₁₋₆ alkyl)amino, C₃₋₆ alkenyl-amino, di(C₃₋₆ alkenyl)amino, substituted C₃₋₆ alkenyl-amino, di(substituted C₃₋₆ alkenyl)amino, or
(iv) pyrrolidino, piperidino, morpholino, imidazolyl, substituted imidazolyl, piperazino, 4-N- C₁₋₆ alkylpiperazino, 4-N-C₃₋₆ alkenylpiperazino, 4-N-(C₁₋₆ alkoxy C₁₋₆ alkyl)piperazino, 4-N-(C₁₋₆ alkoxy C₃₋₆ alkenyl)piperazino, 4-N-(C₁₋₆ alkylamino C₁₋₆ alkyl)piperazino, 4-N-(C₁₋₆ alkylamino C₃₋₆ alkenyl)piperazino,
wherein the substituents are selected from the group consisting of:
(a) hydrogen, hydroxy, halo, trifluoromethyl,
(b) C₁₋₆ alkylalkoxy, C₁₋₆ alkylamino, C₁₋₆ alkylthio,
(c) C₃₋₆ alkenyloxy, C₃₋₆ alkenylamino, C₃₋₆ alkenylthio, or
(d) pyrrolidino, piperidino, morpholino, imidazolyl, substituted imidazolyl, piperazino, 4-N- C₁₋₆ alkylpiperazino, 4-N-C₃₋₆ alkenylpiperazino, 4-N-(C₁₋₆ alkoxy C₁₋₆ alkyl)piperazino, 4-N-(C₁₋₆ alkoxy C₃₋₆ alkenyl)piperazino, 4-N-(C₁₋₆ alkylamino C₁₋₆ alkyl)piperazino, 4-N-(C₁₋₆ alkylamino C₃₋₆ alkenyl)piperazino;
with the proviso that at least one of R₂ and R₃ group be selected from [B (2)] and the phenyl and the substituents be selected from (ii)-( iv) above; or R₂ and R₃ taken together forming an aryl group such as phenyl, pyridyl, in which the aryl may be optionally substituted, wherein the substituents are defined as above in (i)-(iv);
and R₄ is selected from the group consisting of:
(a) hydrogen;
(b) substituted C₁₋₁₁ alkyl or C₂₋₁₁ alkenyl wherein the substituents are independently selected from the group consisting of hydrogen, hydroxy, C₁₋₆ alkyloxy, C₁₋₆alkylthio, C₁₋₆ alkylamino, phenyl-C₁₋₆ alkylamino, C₁₋₆ alkoxycarbonyl and the substituents are selected from (iii)-(iv); or
(c) aryl C₀₋₁₁ alkyl wherein the aryl group is selected from phenyl, imidazolyl, furyl, thienyl,
or its pharmaceutically acceptable salts.

2. Use of a therapeutically effective amount of a A-compound of Formula 1, 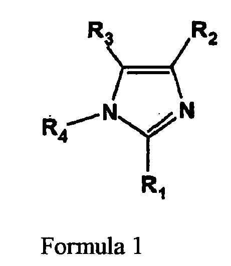 and a pharmaceutically acceptable carrier for the manufacture of a medicament for increasing the sensitivity of tumor cells to an antitumor chemotherapeutic agent, wherein the tumor cells are susceptible to the antitumor chemotherapeutic agent and the tumor cells have become resistant to chemotherapy and further wherein for the compound of Formula 1,the substituents R₁, R₂, R₃, and R₄ are defined as described in A and **B** below:
A. when R₁ is selected from the group consisting of:
(i) substituted C₁₋₁₁ alkyl or substituted C₂₋₁₁ alkenyl, wherein the substituents are selected from the group consisting of hydroxy, C₁₋₆ alkyloxy; or
(ii) mono-, di-, and tri-substituted aryl-C₀₋₁₁ alkyl wherein aryl is selected from the group consisting of phenyl, furyl, thienyl wherein the substituents are selected from the group consisting of:
(a) phenyl, *trans* -2-phenylethenyl, 2-phenylethynyl, 2-phenylethyl, wherein the phenyl group is mono- or disubstituted with a member selected from the group consisting of hydroxy, halo, C₁₋₄ alkyl and C₁₋₄ alkyloxy,
(b) substituted C₁₋₆ alkyl, substituted C₂₋₆ alkyloxy, substituted C₂₋₆ alkylthio, substituted C₂₋₆ alkoxycarbonyl, wherein the substituents are selected from the group consisting of C₁₋₆ alkoxy, and C₁₋₆ alkylthio; and
(c) C₁₋₁₁ CO₂R₅, C₁₋₁₁CONHR₅, *trans*- CH=CHCO₂R₅, or *trans-*CH=CHCONHR₅ wherein R₅ is C₁₋₁₁ alkyl, or phenyl C₁₋₁₁ alkyl, C₁₋₆ alkoxycarbonylmethyleneoxy;
then R₂ and R₃ are each independently selected from the group consisting of mono-, di, and tri-substituted phenyl wherein the substituents are independently selected from:
(i) substituted C₁₋₆ alkyl,
(ii) substituted C₁₋₆ alkyloxy, C₃₋₆ alkenyloxy, substituted C₃₋₆ alkenyloxy,
(iii) substituted C₁₋₆ alkyl-amino, di(substituted C₁₋₆ alkyl)amino,
(iv) C₃₋₆ alkenyl-amino, di(C₃₋₆ alkenyl)amino, substituted C₃₋₆ alkenyl-amino, or di(substituted C₃₋₆ alkenyl)amino,
(v) pyrrolidino, piperidino, morpholino, imidazolyl, substituted imidazolyl, piperazino, 4-N-C₁₋₆ alkylpiperazino, 4-N-C₃₋₆ alkenylpiperazino, 4-N- (C₁₋₆ alkoxy C₁₋₆ alkyl)piperazino, 4-N-(C₁₋₆ alkoxy C₃₋₆ alkenyl)piperazino, 4-N-(C₁₋₆ alkylamino C₁₋₆ alkyl)piperazino, 4-N-(C₁₋₆ alkylamino C₃₋₆ alkenyl)piperazino,
wherein the substituents are selected from the group consisting of
(a) hydroxy, C₁₋₆ alkylalkoxy, C₁₋₆ alkylamino;
(b) C₃₋₆ alkenyloxy, C₃₋₆ alkenylamino, or
(c) pyrrolidino, piperidino, morpholino, imidazolyl, substituted imidazolyl, piperazino, 4-N-C₁₋₆ alkylpiperazino, 4-N-C₃₋₆ alkenylpiperazino, 4-N-(C₁₋₆ alkoxy C₁₋₆ alkyl)piperazino, 4-N-(C₁₋₆ alkoxy C₃₋₆ alkenyl)piperazino, 4-N-(C₁₋₆ alkylamino C₁₋₆ alkyl)piperazino, 4-N-(C₁₋₆ alkylamino C₃₋₆ alkenyl)piperazino,
or R₂ and R₃ taken together forming an aryl group
or substituted aryl, wherein the substituents are defined as above in
(i)-(v);
and R₄ is selected from the group consisting of:
(i) hydrogen;
(ii) substituted C₁₋₁₁ alkyl or C₂₋₁₁ alkenyl wherein the substituents are independently selected from the group consisting of hydrogen, hydroxy, C₁₋₆ alkyloxy, C₁₋₆alkylthio, C₁₋₆ alkylamino, phenyl-C₁₋₆ alkylamino, C₁₋₆ alkoxycarbonyl; or
(iii) substituted aryl C₀₋₁₁ alkyl wherein the aryl group is selected from phenyl, imidazolyl, furyl, thienyl in which the substituents are selected from the group consisting of: A.(a-c); or
B. when R₁ is selected from the group consisting of:
mono-,di-, and tri-substituted aryl-C₀₋₆ alkyl wherein aryl is selected from the group consisting of phenyl, thienyl, and the substituents are selected from the group consisting of:
(a) trans-2-substituted benzimidazolylethenyl, trans-2-substituted benzoxazolylethenyl, or *trans*-2-substituted benzthiazolylethenyl, in which the substituents are selected from the group consisting of hydrogen, hydroxy, halo, trihalomethyl, C₁₋₄ alkyl, C₁₋₄ alkyloxy, C₁₋₄ alkyloxycarbonyl, C₁₋₄ alkylamino, di(C₁₋₄ alkyl)amino, C₃₋₆ alkenylamino, di(C₃₋₆ alkenyl)amino, C₁₋₄ alkyloxy-C₁₋₄ alkylamino, substituted C₁₋₄ alkyl and C₁₋₄ alkyloxy, substituted C₁₋₄ alkyloxycarbonyl, substituted C₁₋₄ alkylamino, di(substituted C₁₋₄ alkyl)amino, substituted C₃₋₆ alkenylamino, di(substituted C₃₋₆ alkenyl)amino, wherein the substituents are as defined above,
(b) *trans*-2-cyano ethenyl, *trans*-2-alkylsulfonyl ethenyl, *trans*-2 - alkenylsulfonyl ethenyl, *trans*-2*-* substituted alkylsulfonyl ethenyl, *trans*-2- substituted alkenylsulfonyl ethenyl, in which the substituents are as defined above,
(c) C₁₋₆ CO₂R₅, *trans*- CH=CHCO₂R₅, C₁₋₆CONHR₅, or *trans-* CH=CHCONHR₅, wherein R₅ is C₁₋₆ alkoxy C₂₋₆ alkyl, amino C₂₋₆ alkyl, C₁₋₆ alkylamino C₂₋₆ alkyl, di(C₁₋₆ alkyl)amino C₂₋₆ alkyl, C₁₋₆ alkylthio C₂₋₆ alkyl, substituted C₁₋₆ alkoxy C₂₋₆ alkyl, substituted C₁₋₆ alkylamino C₂₋₆ alkyl, di(substituted C₁₋₆ alkyl)amino C₂₋₆ alkyl, substituted C₁₋₆ alkylthio C₂₋₆ alkyl, in which the substituents are selected from the group consisting of pyrrolidino, piperidino morpholino, piperazino, **4**-N-C₁₋₆ alkylpiperazino, **4**-N-C₃₋₆ alkenylpiperazino, **4**-N-(C₁₋₆ alkoxy C₁₋₆ alkyl)piperazino, **4**-N-(C₁₋₆ alkoxy C₃₋₆ alkenyl)piperazino, **4**-N-(C₁₋₆ alkylamino C₁₋₆ alkyl)piperazino, **4**-N-(C₁₋₆ alkylamino C₃₋₆ alkenyl)piperazino, imidazolyl, oxazolyl, thiazolyl,
(d) C₁₋₆CONR₆R₇, or *trans-* CH=CHCO₆R₇, wherein R₆ and R₇ are independently selected from the group consisting of C₁₋₆ alkyl, phenyl C₁₋₆ alkyl, C₁₋₆ alkoxycarbonylmethyleneoxy, hydroxy C₂₋₆ alkyl, C₁₋₆ alkyloxy, C₂₋₆ alkyl, amino C₂₋₆ alkyl, C₁₋₆ alkylamino C₂₋₆ alkyl, di(C₁₋₆ alkyl)amino C₂₋₆ alkyl, C₁₋₆ alkylthio C₂₋₆ alkyl, substituted C₁₋₆ alkoxy C₂₋₆ alkyl, substituted C₁₋₆ alkylamino C₂₋₆ alkyl, di(substituted C₁₋₆ alkyl)amino C₂₋₆ alkyl, substituted C₁₋₆ alkylthio C₂₋₆ alkyl, wherein the substituents are selected from the group consisting of pyrrolidino, piperidino, morpholino, piperazino, **4**-N- C₁₋₆ alkylpiperazino, **4**-N-C₃₋₆ alkenylpiperazino, **4**-N-(C₁₋₆ alkoxy C₁₋₆ alkyl)piperazino, **4**-N-(C₁₋₆ alkoxy C₃₋₆ alkenyl)piperazino, **4**-N-(C₁₋₆ alkylamino C₁₋₆ alkyl)piperazino, **4**-N-(C₁₋₆ alkylamino C₃₋₆ alkenyl)piperazino, imidazolyl, oxazolyl, thiazolyl,
(e) R₇ C(O) C₁₋₆ alkyl, R₇ C(O) C₂₋₆ alkenyl, in which R₇ is defined as above [2(d)],
(f) HO-C₁₋₆ alkyl-C₂₋₆ alkenyl, R₇-O-C₁₋₆ alkyl-C₂₋₆ alkenyl, R₇NH-C₁₋₆ alkyl-C₂₋₆ alkenyl, R₆R₇N-C₁₋₆ alkyl-C₂₋₆ alkenyl, R₇NH-C(O)-O-C₁₋₆ akyl-C₂₋₆ alkenyl, R₆R₇N-C(O)-O-C₁₋₆ alkyl-C₂₋₆ alkenyl, R₇O-C(O)-O-C₁₋₆ akyl-C₂₋₆ alkenyl, R₇-C(O)-O-C₁₋₆ alkyl-C₂₋₆ alkenyl, wherein R₆ and R₇ are defined as above [2(d)],
(g) R₇-O-C₀₋₃ akyl-C₃₋₆ cycloalkan-1-yl, R₇NH- C₀₋₃ alkyl- C₃₋₆ cycloalkan-1-yl, R₆R₇N- C₀₋₃ alkyl- C₃₋₆ cycloalkan-1-yl, R₇NH-C(O)-O- C₀₋₃ C₃₋₆ cycloalkan-1-yl, R₆R₇N-C(O)-O- C₀₋₃ alkyl- C₃₋₆ cycloalkan-1-yl, R₇O- C(O)-O- C₀₋₃ alkyl- C₃₋₆ cycloalkan-1-yl, R₇-C(O)-O- C₀₋₃ alkyl- C₃₋₆ cycloalkan- 1 -yl, R₇O-C(O)-C₀₋₃ alkyl- C₃₋₆ cycloalkan-1-yl, wherein **R₇ and R₆** are defined as above [2(d)];
then R₂ and R₃ are each independently selected from the group consisting of:
(1) hydrogen, halo, trihalomethyl, C₁₋₆ alkyl, substituted C₁₋₆ alkyl, C₂₋₆ alkenyl, substituted C₂₋₆ alkenyl, C₁₋₆ alkyloxy, substituted C₁₋₆ alkyloxy, C₃₋₆ alkenyloxy, substituted C₃-₆ alkenyloxy, C₁₋₆ alkylamino, substituted C₁₋₆ alkylamino, C₃₋₆ alkenylamino, substituted C₃₋₆ alkenylamino,
(2) mono-, di-, and tri-substituted phenyl wherein the substituents are independently selected from:
(i) halo, trifluoromethyl, substituted C₁₋₆ alkyl,
(ii) C₁₋₆ alkyloxy, substituted C₁₋₆ alkyloxy, C₃₋₆ alkenyloxy, substituted C₃₋₆ alkenyloxy,
(iii) C₁₋₆ alkyl-amino, di(C₁₋₆ alkyl)amino, substituted C₁₋₆ alkyl-amino, di(substituted C₁₋₆ alkyl)amino, C₃₋₆ alkenyl-amino, di(C₃₋₆ alkenyl)amino, substituted C₃₋₆ alkenyl-amino, or di(substituted C₃₋₆ alkenyl)amino, or
(iv) pyrrolidino, piperidino, morpholino, imidazolyl, substituted imidazolyl, piperazino, **4**-N- C₁₋₆ alkylpiperazino, **4**-N-C₃₋₆ alkenylpiperazino, **4**-N-(C₁₋₆ alkoxy C₁₋₆ alkyl)piperazino, **4**-N-(C₁₋₆ alkoxy C₃₋₆ alkenyl)piperazino, **4**-N-(C₁₋₆ alkylamino C₁₋₆ alkyl)piperazino, **4**-N-(C₁₋₆ alkylamino C₃₋₆ alkenyl)piperazino,
wherein the substituents are selected from the group consisting of:
(a) hydrogen, hydroxy, halo, trifluoromethyl,
(b) C₁₋₆ alkylalkoxy, C₁₋₆ alkylamino, C₁₋₆ alkylthio,
(c) C₃₋₆ alkenyloxy, C₃₋₆ alkenylamino, C₃₋₆ alkenylthio, or
(d) pyrrolidino, piperidino, morpholino, imidazolyl, substituted imidazolyl, piperazino, **4**-N- C₁₋₆ alkylpiperazino, **4**-N-C₃₋₆ alkenylpiperazino, **4**-N-(C₁₋₆ alkoxy C₁₋₆ alkyl)piperazino, **4**-N-(C₁₋₆ alkoxy C₃₋₆ alkenyl)piperazino, **4**--N-(C₁₋₆ alkylamino C₁₋₆ alkyl)piperazino, **4**-N-(C₁₋₆ alkylamino C₃₋₆ alkenyl)piperazino;
with the proviso that at least one of R₂ and R₃ group be selected from [B (2)] and the phenyl and the substituents be selected from (ii)-( iv) above; or R₂ and R₃ taken together forming an aryl group such as phenyl, pyridyl, in which the aryl may be optionally substituted, wherein the substituents are defined as above in (i)-(iv);
and R₄ is selected from the group consisting of:
(a) hydrogen;
(b) substituted C₁₋₁₁ alkyl or C₂₋₁₁ alkenyl wherein the substituents are independently selected from the group consisting of hydrogen, hydroxy, C₁₋₆ alkyloxy, C₁₋₆ alkylthio, C₁₋₆ alkylamino, phenyl-C₁₋₆ alkylamino, C₁₋₆ alkoxycarbonyl and the substituents are selected from (iii)-(iv); or
(c) aryl C₀₋₁₁ alkyl wherein the aryl group is selected from phenyl, imidazolyl, furyl, thienyl ,or its pharmaceutically acceptable salts.
3. Use of a therapeutically effective amount of a compound of Formula 1, 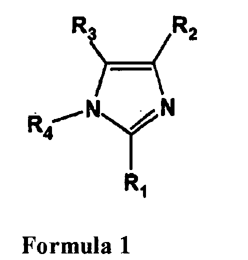 and a pharmaceutically acceptable carrier for the manufacture of a medicament for treatment of tumor cells, wherein the medicament is administered prior to, together with, or subsequent to the administration of an antitumor chemotherapeutic agent,
wherein the compound of Formula 1 has one of the following formulae:
*(formulae of claims 2 to 69 of WO 99*/*02155)*
4. Use of a therapeutically effective amount of a compound of Formula 1, 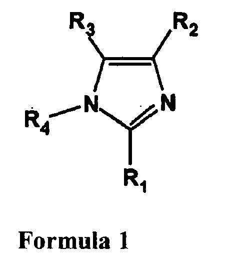 and a pharmaceutically acceptable carrier for the manufacture of a medicament for increasing the sensitivity of tumor cells to an antitumor chemotherapeutic agent, wherein the tumor cells are susceptible to antitumor chemotherapeutic agents and the tumor cells have become resistant to chemotherapy, and further wherein the compound of Formula 1 has one of the following formulae
*(formulae of claims 2 to 69 of WO 99*/*02155)*
5. Use of a therapeutically effective amount of a compound of Formula 1, 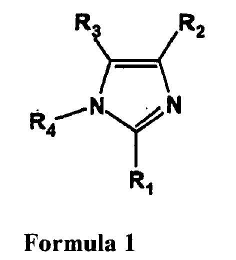 and a pharmaceutically acceptable carrier for the manufacture of a medicament for treatment of tumor cells, wherein the medicament is administered prior to, together with, or subsequent to the administration of an antitumor chemotherapeutic agent,
wherein the compound of Formula 1 has the following formula: 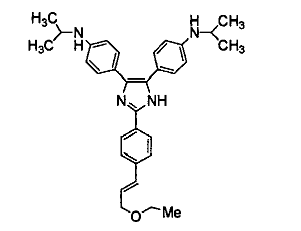 or its pharmaceutically acceptable salts.
6. Use of a therapeutically effective amount of a compound of Formula 1, 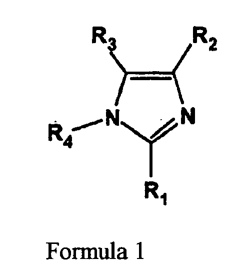 and a pharmaceutically acceptable carrier for the manufacture of a medicament for increasing the sensitivity of tumor cells to an antitumor chemotherapeutic agent, wherein the tumor cells are susceptible to antitumor chemotherapeutic agents and the tumor cells have become resistant to chemotherapy, and further wherein the compound of Formula 1 has the following formula: 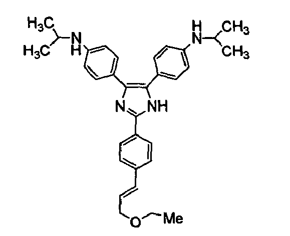 or its pharmaceutically acceptable salts
7.The use according to any one of claims 1, 2, 3, 5 or 6 wherein the antitumor chemotherapeutic agent is Taxol®, vinblastine, vincristine, daunorubicin, or doxorubicin.
8.The use according to claim 4 wherein the antitumor chemotherapeutic agent is Taxol®, vincristine, daunorubicin, or doxorubicin.
9. The use according to any one of 1, 2, 3, 4, 5 or 6 wherein the compound of Formula 1 is administered orally, topically or parenterally.
10. The use according to claim 9 wherein when the compound of Formula 1 is administered parenterally, it is administered via subcutaneous injection, intravenous, intramuscular, intrasternal injection or via infusion techniques.
11. A compound of the following formula: 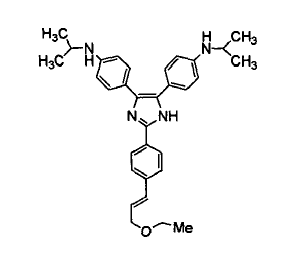 or its pharmaceutically acceptable salts.
12. A pharmaceutical composition comprising a compound of the following formula: 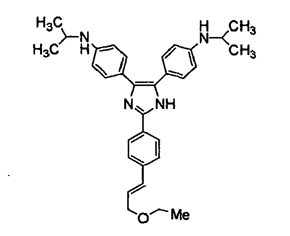 or its pharmaceutically acceptable salts, and a pharmaceutically acceptable carrier.
13. The pharmaceutical composition of claim 12 wherein the pharmaceutical composition is formulated to be suitable for oral, topical or parenteral administration
14. The pharmaceutical composition of claim 12 wherein the pharmaceutical composition is formulated as a suppository for rectal administration.
15. The pharmaceutical composition of claim 13 wherein when the pharmaceutical composition is formulated to be suitable for parenteral administration, it is formulated to be suitable for subcutaneous injection, intravenous, intramuscular, intrasternal injection or infusion.
